# EUROPEAN PATENT SPECIFICATION

(11) **EP 1 750 591 B1**
(45) Date of publication and mention of the grant of the patent: **24.03.2021**
(21) Application number: 05746261.6
(22) Date of filing: 06.05.2005
(51) Int. Cl.: A61B 17/04, A61B 17/064, A61B 17/06, A61B 17/00

(54) **ANCHOR FOR IMPLANTATION IN A BODY**
ANKER FÜR IMPLANTATION IN EINEN KÖRPER
ANCRAGE POUR IMPLANTATION DANS UN CORPS

(30) Priority: 07.05.2004 US 841411; 07.05.2004 US 840951; 07.05.2004 US 840950; 07.05.2004 US 841245; 15.06.2004 US 869472; 14.01.2005 US 36866
(43) Date of publication of application: 14.02.2007
(73) Proprietor: USGI Medical Inc, San Clemente CA 92673 (US)
(72) Inventor: SAADAT, Vahid, Saratoga, CA 95070 (US); MAAHS, Tracy, D., Lake Forest, CA 92679 (US); EWERS, Richard, C., Fullerton, CA 92833 (US); ELMER, Marvin, C., Rancho Santa Margarita, CA 92688 (US); FLORES, Jesus, Perris, CA 92570 (US); KHAIRKHAHAN, Alex, Palo Alto, CA 94301 (US); PEH, Ruey-Feng, Sunnyvale, CA 94087 (US); LAM, Cang, C., Irvine, CA 92612 (US)
(74) Representative: Mathys & Squire
(86) International application number: PCT/US2005/015765
(87) International publication number: WO 2005/110244

(56) References cited:
- EP-A2- 0 755 656
- EP-A2- 1 013 229
- WO-A1-00/30550
- WO-A1-96/20647
- GB-A- 2 337 934
- US-A- 4 006 747
- US-A- 5 507 754
- US-A- 5 626 590
- US-A- 6 152 935
- US-A1- 2002 019 649
- US-A1- 2003 088 250
- US-A1- 2003 167 062
- US-B1- 6 328 758
- US-B1- 6 475 230
- US-B1- 6 692 506

## Description

### BACKGROUND OF THE INVENTION

### Field of the Invention.

The present invention relates to an anchor for implantation in a body.

Morbid obesity is a serious medical condition pervasive in the United States and other countries. Its complications include hypertension, diabetes, coronary artery disease, stroke, congestive heart failure, multiple orthopedic problems and pulmonary insufficiency with markedly decreased life expectancy.

A number of surgical techniques have been developed to treat morbid obesity, e.g., bypassing an absorptive surface of the small intestine, or reducing the stomach size. However, many conventional surgical procedures may present numerous life-threatening post-operative complications, and may cause atypical diarrhea, electrolytic imbalance, unpredictable weight loss and reflux of nutritious chyme proximal to the site of the anastomosis.

Furthermore, the sutures or staples that are often used in these surgical procedures typically require extensive training by the clinician to achieve competent use, and may concentrate significant force over a small surface area of the tissue, thereby potentially causing the suture or staple to tear through the tissue. Many of the surgical procedures require regions of tissue within the body to be approximated towards one another and reliably secured. The gastrointestinal lumen includes four tissue layers, wherein the mucosa layer is the inner-most tissue layer followed by connective tissue, the muscularis layer and the serosa layer.

One problem with conventional gastrointestinal reduction systems is that the anchors (or staples) should engage at least the muscularis tissue layer in order to provide a proper foundation. In other words, the mucosa and connective tissue layers typically are not strong enough to sustain the tensile loads imposed by normal movement of the stomach wall during ingestion and processing of food. In particular, these layers tend to stretch elastically rather than firmly hold the anchors (or staples) in position, and accordingly, the more rigid muscularis and/or serosa layer should ideally be engaged. This problem of capturing the muscularis or serosa layers becomes particularly acute where it is desired to place an anchor or other apparatus transesophageally rather than intraoperatively, since care must be taken in piercing the tough stomach wall not to inadvertently puncture adjacent tissue or organs.

One conventional method for securing anchors within a body lumen to the tissue is to utilize sewing devices to suture the stomach wall into folds. This procedure typically involves advancing a sewing instrument through the working channel of an endoscope and into the stomach and against the stomach wall tissue. The contacted tissue is then typically drawn into the sewing instrument where one or more sutures or tags are implanted to hold the suctioned tissue in a folded condition known as a plication. Another method involves manually creating sutures for securing the plication.

One of the problems associated with these types of procedures is the time and number of intubations needed to perform the various procedures endoscopically. Another problem is the time required to complete a plication from the surrounding tissue with the body lumen. In the period of time that a patient is anesthetized, procedures such as for the treatment of morbid obesity or for GERD must be performed to completion. Accordingly, the placement and securement of the tissue plication should ideally be relatively quick and performed with a minimal level of confidence.

Another problem with conventional methods involves ensuring that the staple, knotted suture, or clip is secured tightly against the tissue and that the newly created plication will not relax under any slack which may be created by slipping staples, knots, or clips. Other conventional tissue securement devices such as suture anchors, twist ties, crimps, etc. are also often used to prevent sutures from slipping through tissue. However, many of these types of devices are typically large and unsuitable for low-profile delivery through the body, e.g., transesophageally.

Moreover, when grasping or clamping onto or upon the layers of tissue with, conventional anchors, sutures, staples, clips, etc., may of these devices are configured to be placed only after the tissue has been plicated and not during the actual plication procedure.

EP1013229 A2 discloses a device for repairing a soft tissue defect, particularly a defect in the meniscus of a knee, comprising an outer wall anchor for engaging against an outside wall of the meniscus on a first side of the defect, and an inner meniscal anchor engaging an inner surface of the meniscus on a second side of the defect.

Documents US2003167062 A1, WO9620647 A1, WO0030550 A1, US6475230 B1, US2002019649 A1, US5626590 A, US2003088250 A1, EP0755656 A2, US5507754 A and GB2337934 A disclose further relevant background art.

### BRIEF SUMMARY OF THE INVENTION

The invention is defined in the appended independent claim 1. Preferred embodiments are disclosed in the dependent claims.

In securing plications which may be created within a body lumen of a patient, various methods and devices may be implemented. Generally, any number of conventional methods may be utilized for initially creating the plication. One method in particular may involve creating a plication through which a tissue anchor may be disposed within or through. A distal tip of a tissue plication apparatus may engage or grasp the tissue and move the engaged tissue to a proximal position relative to the tip of the device, thereby providing a substantially uniform plication of predetermined size. Examples of tools and methods which are particularly suited for delivering the anchoring and securement devices may be seen in further detail in U.S. Pat. App. Serial No. 10/735,030 filed December 12, 2003 (US Patent No. 8,574,243).

In securing these plications, various tissue anchors may be utilized for securing the plications in their configured folds. For example, a plication (or plications) may be secured via a length or lengths of suture extending through the plication and between a distally positioned tissue anchor located on a distal side of the plication and a proximally-positioned tissue anchor located on a proximal side of the plication. Examples of anchors which may be utilized are disclosed in U.S. Pat. App. Serial No. 10/612,170, filed July 1, 2003 (US Publication No. 2004-0122456).

Generally, in securing a tissue plication, a proximally and/or distally located tissue anchor is preferably configured to slide along the connecting suture in a uni-directional manner. For instance, if the proximal anchor is to be slid along the suture, it is preferably configured to translate over the suture such that the tissue plication is cinched between the anchors. In this example, the proximal anchor is preferably configured to utilize a locking mechanism which allows for the free uni-directional translation of the suture therethrough while enabling the anchor to be locked onto the suture if the anchor is pulled, pushed, or otherwise urged in the opposite direction along the suture. This uni-directional anchor locking mechanism facilitates the cinching of the tissue plication between the anchors and it may be utilized in one or several of the anchors in cinching a tissue fold.

Moreover, the types of anchors utilized for the securement of tissue plications are not intended to be limiting. For instance, many of the anchor locking or cinching mechanisms maybe utilized with, e.g., "T"-type anchors as well as with reconfigurable "basket"-type anchors, which generally comprise a number of configurable struts or legs extending between at least two collars or support members. Other variations of these or other types of anchors are also contemplated for use in an anchor locking or cinching assembly.

For instance, linear anchors, i.e., elongate anchors which are configured to fold or become compressed into a bowed or expanded configuration, may also be utilized. Such anchors may be configured in a variety of different configurations, such as flattened ribbon or wire, having one or more openings along its length through which a length of suture may be routed. Generally, a linear-type anchor for placement against a tissue surface may comprise an elongate member having a proximal end, a distal end, and a length therebetween defining a plurality of holes, a length of suture for passage through at least one of the holes, and wherein the elongate member is adapted to be reconfigured from a straightened configuration to an expanded anchoring configuration when the elongate member is compressed longitudinally. In utilizing such a linear-type anchor, one method of positioning the anchor against the tissue surface may generally comprise positioning an elongate member in a straightened configuration against the tissue surface, the elongate member having a proximal end, a distal end, and a length therebetween, and compressing the elongate member longitudinally such that the elongate member reconfigures to an expanded anchoring configuration against the tissue surface.

Furthermore, a single type of anchor may be used exclusively in an anchor locking or cinching assembly; alternatively, a combination of different anchor types each utilizing different anchor locking or cinching mechanisms may be used in a single assembly. Furthermore, the different types of cinching or locking mechanisms are not intended to be limited to any of the particular variations shown and described below but may be utilized in any combinations or varying types of anchors as practicable.

The suture itself may be modified or altered to integrate features or protrusions along its length or a specified portion of its length. Such features may be defined uniformly at regular intervals along the length of suture or intermittently, depending upon the desired locking or cinching effects. Furthermore, the suture may be made from metals such as Nitinol, stainless steels, Titanium, etc., provided that they are formed suitably thin and flexible. Using metallic sutures with the anchoring mechanisms may decrease any possibilities of suture failure and it may also provide a suture better able to withstand the acidic and basic environment of the gastrointestinal system. Also, it may enhance imaging of the suture and anchor assembly if examined under imaging systems. Sutures incorporating the use of features or protrusions along its length as well as sutures fabricated from metallic materials or any other conventional suture type may be utilized with any of the locking or cinching mechanisms described below in various combinations, if so desired.

One variation for utilizing a locking mechanism which allows for free uni-directional translation of the suture through the anchor may include blocks or members which are adapted to slide within or upon an anchor to lock the suture. These blocks or members may include tapered edges which act to cleat the suture depending upon the direction the anchor is translated relative to the suture. Moreover, these blocks may be biased or urged to restrict the movement of the suture using a variety of biasing elements, such as springs, etc. In addition to blocks, one or several locking tabs which are levered to allow uni-directional travel of the suture through an anchor may also be utilized.

Aside from the use of mechanical locking features integrated within or with the anchor bodies, locking mechanisms may also utilize a variety of knotting techniques. Conventional knots, which are typically tied by the practitioner either within the body or outside the body and advanced over the suture length, may be utilized for locking the anchor in place relative to the tissue fold and opposing anchor; however, self-locking knots which enable the uni-directional travel of an anchor body relative to the suture and tissue are desirable. Accordingly, many different types of self-locking knots may be advanced with the anchor over the suture such that translation along a distal direction is possible yet reverse translation of the anchor is inhibited.

Various anchor cinching or locking mechanisms utilizing friction as a primary source for locking may also be implemented. For instance, locking pins may be urged or pushed into a frictional interference fit with portions or areas of the suture against the anchor or portions of the anchor. The use of such pins may effectively wedge the suture and thereby prevent further movement of the anchor along the suture length. In addition to pins, locking collars or collets may also be used to cinch or lock the suture.

In addition to friction-based locking and cinching mechanisms utilizable in tissue anchors, other mechanisms which create tortuous paths for the suture within or through the anchors may also be utilized for creating uni-directional locking. One cinching variation may utilize a pulley or pin contained within the anchor over which a portion of the suture may travel. The looped suture may then be routed proximally and secured with a slip knot. As tension is applied to the suture, the slip knot may prevent the further movement of the anchor relative to the suture.

Another variation on utilizing tortuous paths may comprise collars which are independent from or integrally formed with the anchors. Such cinching collars may generally be formed into tubular structures having obstructions or interference elements disposed or formed within the collar lumen where the obstructions may, for example, be formed from portions of the cinching collar itself. These obstructions may be adapted to form upon releasing of a constraining force when the anchor is to be locked into position. Alternatively, the interference elements or obstructions may comprise separate elements disposed within the collar lumen, such as one or more balls, spheres, etc. These obstructions or elements may be used to form a tortuous path through which the suture may be routed to lock the suture within.

Moreover, locking collars which form tortuous paths may be adapted to reconfigure themselves from a constrained delivery configuration to a deployed locking configuration when the anchor is to be cinched or locked into position relative to the tissue and suture. The locking collars may be configured to take various configurations, such as a proximally extending "S"-type, or other types, configuration.

Other cinching and locking mechanisms which utilize mechanical clamping or crimping to achieve locking of the suture within or through the anchors may also be used to facilitate uni-directional locking. For instance, a simple mechanical crimp may be fastened upon the suture proximally of the anchor to prevent the reverse motion of the anchor. The crimp may be a simple tubular member or it may be integrally formed onto a proximal portion of the anchor body itself.

Aside from the crimping mechanisms described above, additional measures may be optionally implemented to facilitate the cinching or locking of an anchor. Other measures may also be taken to inhibit any damage from occurring to the suture routed through an anchor. For instance, to ensure that the integrity of the suture is maintained in the presence of metallic basket anchors and to ensure that the suture is not subjected to any nicks or cuts, the portion of the suture passing through basket anchor may be encased in a protective sleeve made, e.g., from polypropylene, PTFE, etc.

Another measure which may optionally be implemented are cinching or locking mechanisms which take advantage of any cold-flow effects of an engaged portion of suture by the tissue anchor. For instance, if a portion of the suture is wedged against the collar of an anchor or cinching member to lock the anchor, the portion of the collar may have multiple holes defined over its surface to allow for portions of the engaged suture to cold-flow at least partially into or through the holes to enhance the locking effects.

Alternatively, the collar may be formed with an electrically conductive inner sleeve surrounded by an outer sleeve capable of flowing at least partially when heated. The inner sleeve may have a number of holes defined over its surface such that when the outer sleeve is heated, either by inductive heating or any other method, the outer sleeve material may flow through the holes and into contact with the suture passing therethrough. This contact may also enhance the locking effects of the collar.

### BRIEF DESCRIPTION OF THE DRAWINGS

Fig. 1A shows a side view of one variation of a tissue plication apparatus which may be used to create tissue plications and to deliver cinching or locking anchors into the tissue.
Figs. 1B and 1C show detail side and perspective views, respectively, of the tissue manipulation assembly of the device of Fig. 1A.
Figs. 2A to 2D show an example of a tissue plication procedure for the delivery and placement of tissue anchors.
Figs. 3A to 3G show detail cross-sectional views of an anchor delivery assembly in proximity to a tissue plication and an example of delivering the tissue anchors on distal and proximal sides of the plication.
Figs. 4A and 4B show side and end views, respectively, of one anchor variation which is illustrated in the form of a T-type anchor utilizing locking blocks or members for cinching and locking the suture.
Fig. 5 shows a side view of another cinching anchor variation utilizing locking blocks or members.
Fig. 6 shows yet another side view of a cinching anchor variation utilizing locking blocks or members.
Fig. 7 shows a perspective view of another locking anchor variation in which the anchor body defines an opening having a tapered or grooved portion.
Figs. 8A and 8B show cross-sectional side and top views, respectively, of another locking anchor variation utilizing a through-hole passage or opening and uni-directional levers or pivots through which the suture may pass.
Fig. 8C shows a cross-sectional side view of an anchor body in combination with a modified suture having integrated features or protrusions defined along its length.
Figs. 9A and 9B show cross-sectional views of locking anchor variations having biased locking members in combination with a knotted suture.
Fig. 9C shows another modification of the suture which may be coated with a metallic covering or slid within a sleeve.
Fig. 10 shows a cross-sectional side view of an anchor assembly which utilizes a choke-type loop for cinching the anchors uni-directionally towards one another.
Fig. 11A shows a perspective view of another anchor assembly utilizing a slip knot at the proximal anchor.
Figs. 11B and 11C show top and cross-sectional side views, respectively, of an anchor which may optionally define grooves or channels extending at least partially therein to facilitate the cinching or wedging of the sutures within the grooves.
Figs. 12A to 12G show examples of anchor assemblies utilizing various slip knots and looped sections which provide uni-directional travel for the anchors over the sutures.
Fig. 13A shows a cross-sectional side view of an anchor delivery system delivering a basket-type anchor into or through a tissue plication.
Fig. 13B shows a cross-sectional side view of multiple tissue plications which may be cinched towards one another and basket anchors as being deliverable through one or both tissue plications.
Figs. 14A and 14B show cross-sectional side views of an anchor cinching assembly utilizing a cinching collar or collet which may be wedged into an anchor collar for clamping upon the suture.
Figs. 15A and 15C show cross-sectional side views of another anchor cinching assembly utilizing a pin for wedging against a portion of the suture.
Figs. 15B and 15D show end views of the assembly of Figs. 15A and 15C, respectively.
Fig. 15E shows a perspective view of another cinching variation utilizing one or more tapered pins or blocks slidably disposed within a tapered channel defined in a proximal collar of the anchor.
Fig. 15F shows a perspective view of the tapered pins from Fig. 15E.
Figs. 15G and 15H show cross-sectional side views of an alternative cinching assembly having a retractable pin in an engaged and disengaged configuration, respectively.
Figs. 16A and 16B show cross-sectional side views of another variation of a cinching assembly having a rotatable cinching collar.
Figs. 17A and 17B show cross-sectional side views of another cinching assembly having a retaining tube for providing a counterforce to stabilize the assembly during cinching or locking.
Figs. 18A and 18B show cross-sectional side views of another cinching assembly having one or several biasing members or cinching tabs.
Figs. 18C and 18D show end and perspective views, respectively, of a suture release member which may be used with the assembly of Figs. 18A and 18B.
Figs. 19A and 19B show cross-sectional side views of another variation of a cinching assembly utilizing a deformable cinching member positioned within the anchor and distally of the anchor collar.
Fig. 20A shows a cross-sectional side view of another cinching assembly utilizing a pivoting cinching member configured to lock against the suture.
Figs. 20B, 20C, and 20D show end and cross-sectional side views, respectively, of the pivoting member positioned within the anchor collar.
Figs. 20E and 20F show cross-sectional side and perspective views, respectively, of another cinching assembly having a pivoting cinching member positioned proximally of the anchor collar.
Figs. 21A and 21B show cross-sectional side views of another cinching assembly configured to cinch or lock the suture with a tapered collar.
Fig. 22A shows a cross-sectional side view of another cinching assembly utilizing a looped suture and a slip knot for cinching the anchor over the suture.
Figs. 22B and 22C show cross-sectional side and detail views, respectively, of another cinching assembly which may be utilized with a portion of suture wrapped or looped about a pin which enables uni-directional travel of the anchor relative to the suture
Figs. 22D and 22E show cross-sectional side and detail views, respectively, of another cinching assembly utilizing looped suture wedged within the anchor collar.
Fig. 23 shows a cross-sectional side view of a cinching assembly variation utilizing a number of pulleys to create the cinching effect.
Fig. 24A shows a cross-sectional side view of another cinching assembly variation in which a cinching sleeve may be used to create a tortuous path for the suture.
Figs. 24B and 24C show cross-sectional side views of another cinching assembly variation having a tubular structure, with and without retaining arms, respectively, positioned within the anchor collar through which the suture may pass uni-directionally.
Fig. 24D shows a perspective view of one variation of the tubular structure of Fig. 24C with retaining arms.
Figs. 25A and 25B show cross-sectional side views of another cinching assembly variation in which a cinching collar, which may be independent of the anchor or formed integrally with the anchor, respectively, may have a tortuous path formed within the collar.
Fig. 25C shows a perspective view of the collar of Fig. 25A in its unobstructed configuration with a constraining sleeve which may be positioned within the collar.
Figs. 26A and 26B show cross-sectional side views of another cinching assembly variation utilizing one or several pivoting levers which allow uni-directional travel of the suture therethrough.
Figs. 26C and 26D show alternative end views of the assembly of Fig. 26A in which the lever may be configured to prevent over cinching onto the suture.
Figs. 26E to 26G show cross-sectional side views of alternative cinching assemblies in which the levers may be variously configured to create the tortuous path.
Figs. 27A and 27B show side views of another cinching assembly variation in a delivery profile and a reconfigured profile, respectively, which utilizes a crimp which may be self-forming.
Figs. 28A and 28B show cross-sectional side views of another cinching assembly variation utilizing either two cinching collars or a single integral cinching collar, respectively.
Fig. 28C shows a cross-sectional side view of the cinching collar of Fig. 28A in one configuration for cinching the suture.
Figs. 28D and 28E show perspective views of the cinching collar of Fig. 28A in a delivery profile and a reconfigured profile.
Fig. 28F shows a cross-sectional side view of another variation for a cinching configuration of the cinching collar of Fig. 28B.
Figs. 28G and 28H show cross-sectional side views of another cinching assembly variation in a delivery profile and reconfigured profile, respectively, in which an elongate cinching member may reconfigure itself to create a tortuous path for the suture.
Figs. 29A and 29B show cross-sectional side views of another cinching assembly variation utilizing a mechanical crimp.
Figs. 30A and 30B show cross-sectional side views of another cinching assembly variation in which a mechanical crimp may be utilized on the proximal collar of the anchor body.
Fig. 31A shows a cross-sectional side view of a variation of a tool assembly which may be adapted to apply a mechanical crimping force upon a crimping collar.
Figs. 31B to 31D show side, end, and perspective views, respectively, of a variation on a crimping collar which may be utilized as a separate crimping sleeve or as part of the anchor collar.
Figs. 32A and 32B show cross-sectional side and perspective views, respectively, of an alternative crimping tool.
Figs. 33A and 33B show perspective and end views, respectively, of a representative basket anchor having a protective sleeve encasing the suture disposed within the anchor.
Figs. 34A and 34B show cross-sectional side and perspective views, respectively, of a cinching collar defining a plurality of holes through the surface of the collar for enhancing the locking effects with the suture.
Fig. 35A shows a cross-sectional side view of a cinching assembly variation which may utilize inductive heating to partially melt a portion of an outer sleeve into contact with the suture to enhance the anchor locking effects.
Figs. 35B and 35C show perspective assembly and exploded views, respectively, of an electrically conductive inner sleeve contained within the outer sleeve.
Figs. 35D and 35E show perspective views of alternative inner sleeves which may be utilized with the assembly of Fig. 35A.
Figs. 36A and 36B show perspective views of variations of linear anchors generally comprised of elongate ribbon or flattened wire and defining one or more openings along their lengths.
Figs. 37A and 37B show the ribbon anchors of Figs. 36A and 36B, respectively, with lengths of suture routed through the openings.
Fig. 38 shows another variation of a ribbon anchor having alternating portions of the ribbon material between the openings notched out or removed.
Fig. 39 shows another variation of a ribbon anchor defining undulations such that an "S"-type ribbon pattern is formed.
Fig. 40A shows the ribbon anchor of Fig. 36A with biasing springs positioned along portions of the ribbon anchor with suture routed therethrough.
Fig. 40B shows another variation of the ribbon anchor of Fig. 40A having biased ribbon elements rather than springs.
Fig. 40C shows the ribbon anchor of Fig. 40A in a partially collapsed configuration.
Fig. 40D shows the ribbon anchor of Fig. 40B in a partially collapsed configuration.
Fig. 41 shows an alternative variation of the ribbon anchor comprised of a tubular member.
Fig. 42 shows a perspective view of another alternative variation of the ribbon anchor comprised of a tubular member having a partial cut-out along its length.
Fig. 43 shows a perspective view of yet another alternative variation of the ribbon anchor having multiple cut-outs along its length.
Fig. 44 shows a perspective view of yet another alternative having multiple individual lengths of elements encased (or at least partially encased) in a coating or covering.
Fig. 45 shows a perspective view of yet another alternative having one or more lengths of wire covered or coated.
Fig. 46 shows another variation in which a length of the ribbon anchor may have a non-uniform thickness.
Figs. 47A and 47B shows yet other variations in which a length of wire having eyelets or defining loops may be utilized to fold or flatten into an expanded pattern.
Fig. 48 shows another variation in which a wire or ribbon may be comprised of a shape memory alloy which is configured to form a tangled portion of the wire when unconstrained.
Figs. 49A and 49B show partial cross-sectional views of ribbon anchors in a flattened linear configuration becoming compressed into a collapsed and expanded configuration, respectively, against a fold of tissue.
Figs. 50A to 50F show top views of various bowed or expanded configurations of a ribbon anchor.
Figs. 51A and 51B are side-views, partially in section, of a cinching assembly comprising an interference element.
Figs. 52A-52C are, respectively, an isometric view, a sectional isometric view and a side-sectional view of the cinching assembly of Figs. 51.
Figs. 53A-53D are side-views, partially in section, of variations of the cinching assembly of Figs. 51 and 52.

### DETAILED DESCRIPTION OF THE INVENTION

In order to first create the plication within a body lumen of a patient, various methods and devices may be implemented. The anchoring and securement devices may be delivered and positioned via an endoscopic apparatus that engages a tissue wall of the gastrointestinal lumen, creates one or more tissue folds, and disposes one or more of the anchors through the tissue fold(s). The tissue anchor(s) may be disposed through the muscularis and/or serosa layers of the gastrointestinal lumen.

Generally, in creating a plication through which a tissue anchor may be disposed within or through, a distal tip of a tissue plication apparatus may engage or grasp the tissue and move the engaged tissue to a proximal position relative to the tip of the device, thereby providing a substantially uniform plication of predetermined size.

Formation of a tissue fold may be accomplished using at least two tissue contact areas that are separated by a linear or curvilinear distance, wherein the separation distance between the tissue contact points affects the length and/or depth of the fold. In operation, a tissue grabbing assembly engages or grasps the tissue wall in its normal state (i.e., non-folded and substantially flat), thus providing a first tissue contact area. The first tissue contact area then is moved to a position proximal of a second tissue contact area to form the tissue fold. The tissue anchor assembly then may be extended across the tissue fold at the second tissue contact area. Optionally, a third tissue contact point may be established such that, upon formation of the tissue fold, the second and third tissue contact areas are disposed on opposing sides of the tissue fold, thereby providing backside stabilization during extension of the anchor assembly across the tissue fold from the second tissue contact area.

The first tissue contact area may be utilized to engage and then stretch or rotate the tissue wall over the second tissue contact area to form the tissue fold. The tissue fold may then be articulated to a position where a portion of the tissue fold overlies the second tissue contact area at an orientation that is substantially normal to the tissue fold. A tissue anchor may then be delivered across the tissue fold at or near the second tissue contact area. An apparatus in particular which is particularly suited to deliver the anchoring and securement devices described herein may be seen in further detail in U.S. Pat. App. Serial No. 10/735,030 filed December 12, 2003 and entitled "Apparatus And Methods For Forming And Securing Gastrointestinal Tissue Folds" (US Patent No. 8,574,243).

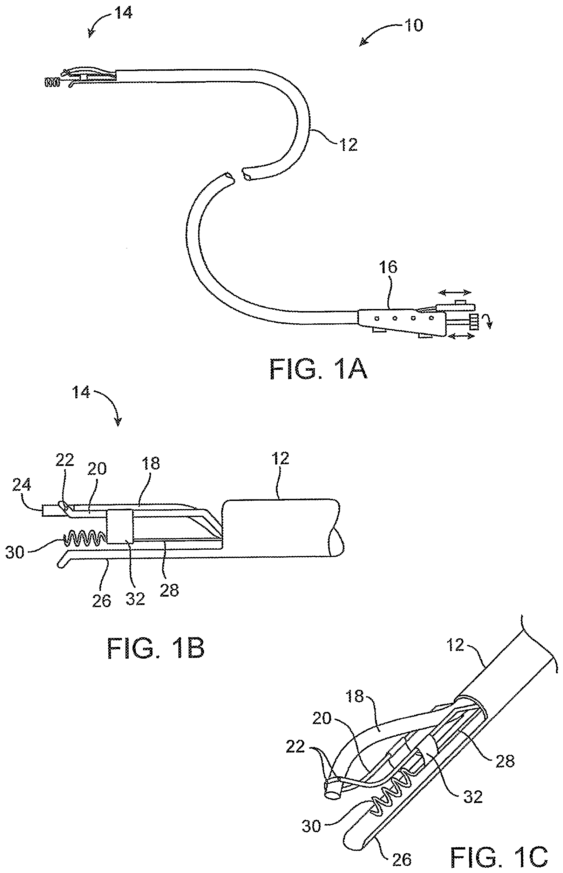

An illustrative side view of a tissue plication assembly 10 which may be utilized with the tissue anchors described herein is shown in Fig. 1A. The plication assembly 10 generally comprises a catheter or tubular body 12 which may be configured to be sufficiently flexible for advancement into a body lumen, e.g., transorally, percutaneously, laparoscopically, etc. Tubular body 12 may be configured to be torqueable through various methods, e.g., utilizing a braided tubular construction, such that when handle 16 is manipulated and rotated by a practitioner from outside the body, the torquing force is transmitted along body 12 such that the distal end of body 12 is rotated in a corresponding manner.

Tissue manipulation assembly 14 is located at the distal end of tubular body 12 and is generally used to contact and form the tissue plication, as mentioned above. Fig. 1B shows an illustrative detail side view of tissue manipulation assembly 14 which shows launch tube 18 extending from the distal end of body 12 and in-between the arms of upper extension member or bail 20. Launch tube 18 may define launch tube opening 24 and may be pivotally connected near or at its distal end via hinge or pivot 22 to the distal end of upper bail 20. Lower extension member or bail 26 may similarly extend from the distal end of body 12 in a longitudinal direction substantially parallel to upper bail 20. Upper bail 20 and lower bail 26 need not be completely parallel so long as an open space between upper bail 20 and lower bail 26 is sufficiently large enough to accommodate the drawing of several layers of tissue between the two members.

Upper bail 20 is shown in the figure as an open looped member and lower bail 26 is shown as a solid member; however, this is intended to be merely illustrative and either or both members may be configured as looped or solid members. Tissue acquisition member 28 may be an elongate member, e.g., a wire, hypotube, etc., which terminates at a tissue grasper 30, in this example a helically-shaped member, configured to be reversibly rotatable for advancement into the tissue for the purpose of grasping or acquiring a region of tissue to be formed into a plication. Tissue acquisition member 28 may extend distally from handle 16 through body 12 and distally between upper bail 20 and lower bail 26. Acquisition member 28 may also be translatable and rotatable within body 12 such that tissue grasper 30 is able to translate longitudinally between upper bail 20 and lower bail 26. To support the longitudinal and rotational movement of acquisition member 28, an optional guide or sled 32 may be connected to upper 20 or lower bail 26 to freely slide thereon. Guide 32 may also be slidably connected to acquisition member 28 such that the longitudinal motion of acquisition member 28 is supported by guide 32.

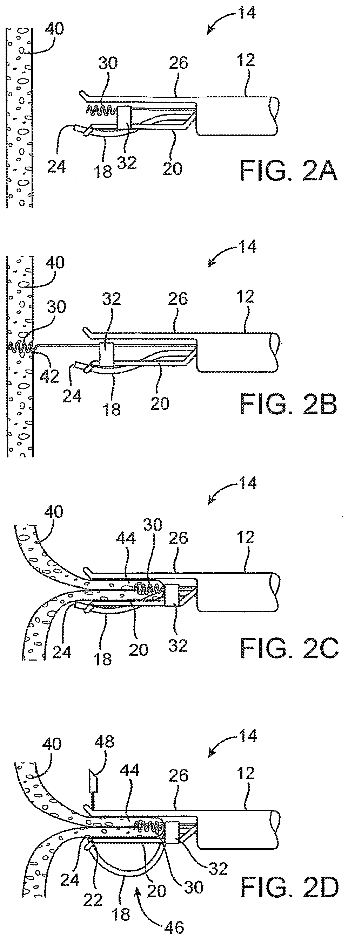

An example of a tissue plication procedure is seen in Figs. 2A to 2D for delivering and placing a tissue anchor and is disclosed in further detail in U.S. Pat. App. Serial No. 10/735,030 filed December 12, 2003 (US Patent No. 8,574,243). Tissue manipulation assembly 14, as seen in Fig. 2A, may be advanced into a body lumen such as the stomach and positioned adjacent to a region of tissue wall 40 to be plicated. During advancement, launch tube 18 may be configured in a delivery profile such that tube 18 is disposed within or between the arms of upper bail 20 to present a relatively small profile.

Once tissue manipulation assembly 14 has been desirably positioned relative to tissue wall 40, tissue acquisition member 30 may be advanced distally such that tissue acquisition member 30 comes into contact with tissue wall 40 at acquisition location or point 42. As acquisition member 30 is distally advanced relative to body 12, guide 32, if utilized, may slide distally along with member 30 to aid in stabilizing the grasper. If a helically shaped acquisition member 30 is utilized, as illustrated in Fig. 2B, it may be rotated from its proximal end at handle 16 and advanced distally until the tissue at point 42 has been firmly engaged by acquisition member 30. This may require advancement of acquisition member 30 through the mucosal layer and at least into or through the underlying muscularis layer and preferably into or through the serosa layer.

The grasped tissue may then be pulled proximally between upper 20 and lower bails 26 via acquisition member 30 such that the acquired tissue is drawn into a tissue fold 44, as seen in Fig. 2C. As acquisition member 30 is withdrawn proximally relative to body 12, guide 32 may also slide proximally to aid in stabilizing the device especially when drawing the tissue fold 44.

Once the tissue fold **44** has been formed, launch tube **18** may be advanced from its proximal end at handle **16** such that a portion **46** of launch tube **18,** which extends distally from body **12,** is forced to rotate at hinge or pivot **22** and reconfigure itself such portion **46** forms a curved or arcuate shape that positions launch tube opening **24** perpendicularly relative to a longitudinal axis of body **12** and/or bail members **20, 26.** Launch tube **18,** or at least portion **46** of launch tube **18,** is preferably fabricated from a highly flexible material or it may be fabricated, e.g., from Nitinol tubing material which is adapted to flex, e.g., via circumferential slots, to permit bending. Alternatively, assembly **14** may be configured such that launch tube **18** is reconfigured simultaneously with the proximal withdrawal of acquisition member **30** and acquired tissue **44.**

As discussed above, the tissue wall of a body lumen, such as the stomach, typically comprises an inner mucosal layer, connective tissue, the muscularis layer and the serosa layer. To obtain a durable purchase, e.g., in performing a stomach reduction procedure, the staples or anchors used to achieve reduction of the body lumen are preferably engaged at least through or at the muscularis tissue layer, and more preferably, the serosa layer. Advantageously, stretching of tissue fold **44** between bail members **20, 26** permits an anchor to be ejected through both the muscularis and serosa layers, thus enabling durable gastrointestinal tissue approximation.

As shown in Fig. 2D, once launch tube opening **24** has been desirably positioned relative to the tissue fold **44,** needle assembly **48** may be advanced through launch tube **18** via manipulation from its proximal end at handle **16** to pierce preferably through a dual serosa layer through tissue fold **44.** Needle assembly **48** is preferably a hollow tubular needle through which one or several tissue anchors may be delivered through and ejected from in securing the tissue fold **44,** as further described below.

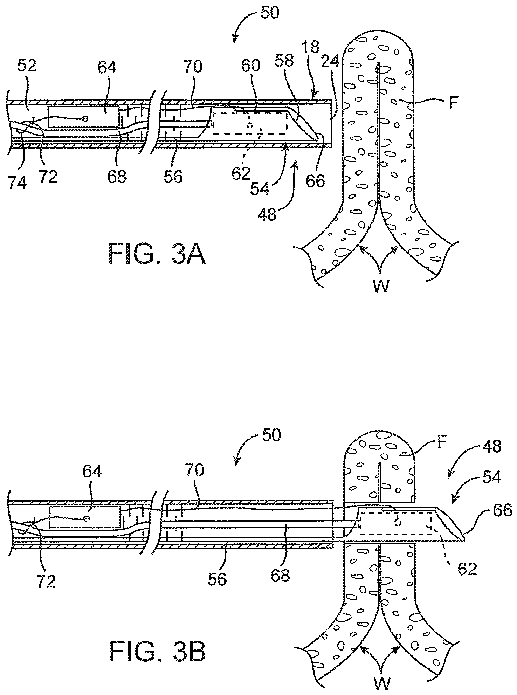

Because needle assembly **48** penetrates the tissue wall twice, it exits within the body lumen, thus reducing the potential for injury to surrounding organs. A detail cross-sectional view is shown in Fig. 3A of anchor delivery assembly **50** in proximity to tissue fold **F.** In this example, tissue fold **F** may comprise a plication of tissue created using the apparatus **10** described herein or any other tool configured to create such a tissue plication. Tissue fold **F** may be disposed within a gastrointestinal lumen, such as the stomach, where tissue wall **W** may define the outer or serosal layer of the stomach. Anchor delivery assembly may generally comprise launch tube **18** and needle assembly **48** slidingly disposed within launch tube lumen **52.** Needle assembly **48** is generally comprised of needle **54,** which is preferably a hollow needle having a tapered or sharpened distal end **66** to facilitate its travel into and/or through the tissue. Other parts of the assembly, such as upper and lower bail members **20, 26,** respectively, and tissue acquisition member **28** have been omitted from these figures only for clarity.

Once launch tube **18** has been desirably positioned with respect to tissue fold **F,** needle **54** may be urged or pushed into or through tissue fold **F** via needle pushrod or member **56** from its proximal end preferably located within handle **16.** Needle **54** may define needle lumen **58** within which distal anchor **62** and/or proximal anchor **64** may be situated during deployment and positioning of the assembly. A single suture or flexible element **70** (or multiple suture elements) may connect proximal anchor **64** and distal anchor **62** to one another. For instance, element **70** may comprise various materials such as monofilament, multifilament, or any other conventional suture material, elastic or elastomeric materials, e.g., rubber, etc.

Alternatively, metals which are biocompatible may also be utilized for suture materials. For instance, sutures may be made from metals such as Nitinol, stainless steels, Titanium, etc., provided that they are formed suitably thin and flexible. Using metallic sutures with the anchoring mechanisms described herein may additionally provide several benefits. For example, use of metallic suture material may decrease any possibilities of suture failure due to inadvertent cutting or shearing of the suture, it may provide a suture better able to withstand the acidic and basic environment of the gastrointestinal system, and it may also enhance imaging of the suture and anchor assembly if examined under conventional imaging systems such as X-rays, fluoroscopes, MRI, etc. As used herein, suture **70** may encompass any of these materials or any other suitable material which is also biocompatible.

Needle **54** may optionally define needle slot **60** along its length to allow suture **70** to pass freely within and out of needle **54** when distal anchor **62** is ejected from needle lumen **58.** Alternatively, rather than utilizing needle slot **60,** needle **54** may define a solid structure with suture **70** being passed into needle lumen **58** via the distal opening of needle **54.**

The proximal end of suture **70** may pass slidingly through proximal anchor **64** to terminate in suture loop **74** via cinching knot **72.** Suture loop **74** may be omitted and the proximal end of suture **70** may terminate proximally of the apparatus **10** within control handle **16,** proximally of control handle **16,** or at some point distally of control handle **16.** In this variation, suture loop **74** may be provided to allow for a grasping or hooking tool to temporarily hold suture loop **74** for facilitating the cinching of proximal **64** and distal **62** anchors towards one another for retaining a configuration of tissue fold **F,** as described in further detail below. Cinching knot **72** may also comprise a slidable knot which may be slid distally along suture **70** to lock or hold against proximal anchor **64** once the tissue fold **F** and anchors **62, 64** have been desirably positioned and tensioned, as also described below in further detail.

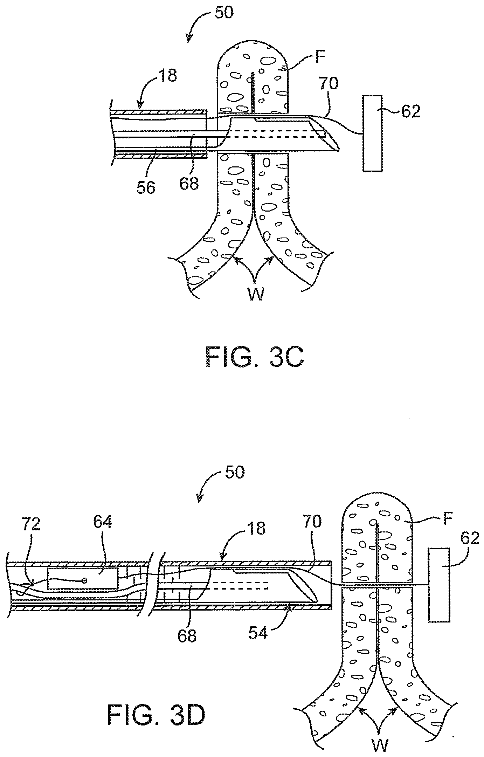

After needle assembly **48** has been pushed distally out through launch tube opening **24** and penetrated into and/or through tissue fold **F,** as shown in Fig. 3B, anchor pushrod or member **68** may be actuated also via its proximal end to eject distal anchor **62,** as shown in Fig. 3C. Once distal anchor **62** has been ejected distally of tissue fold **F,** Fig. 3D shows how needle **54** may be retracted back through tissue fold **F** by either retracting needle **54** back within launch tube lumen **52** or by withdrawing the entire anchor delivery assembly **50** proximally relative to tissue fold **F.**

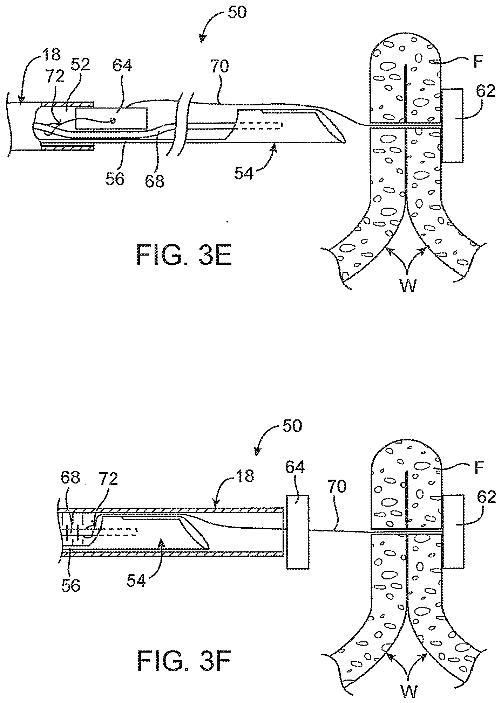

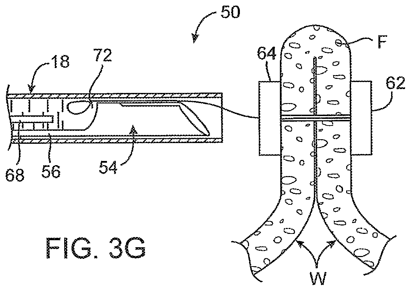

Fig. 3E shows that once needle **54** has been retracted, proximal anchor **64** may then be ejected from launch tube **18** on a proximal side of tissue fold **F.** With both anchors **62, 64** disposed externally of launch tube **18** and suture **70** connecting the two, proximal anchor **64** may be held against the distal end of launch tube **18** and urged into contact against tissue fold **F,** as shown in Figs. 3F and 3G, respectively. As proximal anchor **64** is urged against tissue fold **F,** proximal anchor **64** or a portion of suture **70** may be configured to provide any number of directionally translatable locking mechanisms which provide for movement of an anchor along suture **70** in a first direction and preferably locks, inhibits, or prevents the reverse movement of the anchor back along suture **70.** In other alternatives, the anchors may simply be delivered through various elongate hollow tubular members, e.g., a catheter, trocars, etc.

With respect to the anchor assemblies described herein, the types of anchors shown and described are intended to be illustrative and are not limited to the variations shown. For instance, several of the tissue anchor variations are shown as "T"-type anchors while other variations are shown as reconfigurable "basket"-type anchors, which may generally comprise a number of configurable struts or legs extending between at least two collars or support members. Other variations of these or other types of anchors are also contemplated for use in an anchor assembly. Examples of anchors which may be utilized are disclosed in co-pending U.S. Pat. App. Serial No. 10/612,170, filed July 1, 2003 (US Publication No. 2004-0122456). Moreover, a single type of anchor may be used exclusively in an anchor assembly; alternatively, a combination of different anchor types may be used in an anchor assembly. Furthermore, the different types of cinching or locking mechanisms are not intended to be limited to any of the particular variations shown and described but may be utilized in any of the combinations or varying types of anchors as practicable.

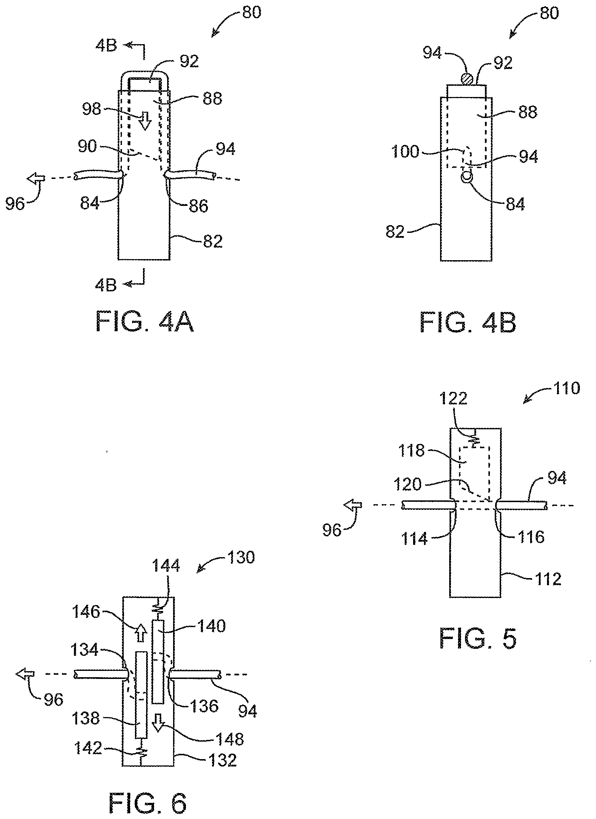

To accomplish the secure placement of anchors having uni-directional anchor movement over the suture in a self-locking manner, various devices and methods may be utilized. Figs. 4A and 4B show side and end views, respectively, of one anchor variation 80 which is illustrated in the form of a T-type anchor. Although a T-type anchor is shown, the methods and devices used to cinch the anchor may be utilized in other types of anchors, which will be described below. Variation 80 may generally comprise an anchor body 82 having a circular, rectangular, square, etc., cross-section which defines openings 84 and 86 on opposing sides of the anchor 80. Locking block or member 88 may be slidably disposed within anchor body 82 and define a tapered face 90 on the side of block 88 which tapers to at least one of the openings, in this case opening 86. Openings 84, 86 are preferably aligned with one another although this is not necessary.

Suture 94 may be routed through opening 84, around locking block 88, and back out through opening 86 such that when anchor body 82 is translated in the direction of arrow 96, anchor body 82 may slide freely over suture 94 due to the manner of tapered face 90 contacting suture 84 within opening 84. However, if anchor body 82 were translated in the opposite direction, tension within suture 94 may pull locking block 88 via suture 94 placed over contact surface 92 such that when block 88 translates in the direction of arrow 98, suture 94 at opening 86 is forced into groove 100 defined along the leading edge of block 88, as shown in Fig. 4B. This cleating action may effectively inhibit or prevent any further movement of anchor body 82 over suture 94. Accordingly, anchor body 82 may be moved uni-directionally relative to suture 94 and a distally located anchor to effectively cinch tissue therebetween.

Fig. 5 illustrates another cinching anchor in the side view of anchor variation 110. In this variation, anchor body 112 similarly defines openings 114 and 116 through which suture 96 may be routed. Locking block or member 118, which may similarly also define tapered face 120 may be slidably disposed within anchor body 112. Locking block 118 may be urged via a biasing member, for instance spring **122,** to maintain a biasing force against suture **94** passing through anchor body **112.** As anchor body **112** is translated over suture **94** in the direction of arrow **96,** tapered face **120** may allow suture **94** to pass freely between openings **114, 116.** However, if anchor body **112** were to be moved in the opposite direction, biasing member **122** may force locking block **118** to exert a force at its leading edge against suture **94,** thereby preventing its movement and allowing only uni-directional movement.

Yet another locking anchor variation **130** is shown in the side view in Fig. 6. In this variation, anchor body **132** also defines openings **134,136** through which suture **94** may pass. Within anchor body **132,** multiple locking blocks or members **138,140** may be configured to become biased in opposing directions via biasing members or springs **142, 144,** respectively. Each of locking blocks **138, 140** may define an opening through which suture **94** may pass. Thus, when anchor body **132** is slowly moved over suture **94** in a first direction, the anchor may translate freely. However, when moved quickly in the opposite direction, the biasing members **142, 144** may urge their respective locking blocks **138, 140** in directions **146, 148** to create a tortuous path through the blocks and inhibit or prevent the reverse movement of anchor body **132** relative to suture **94.**

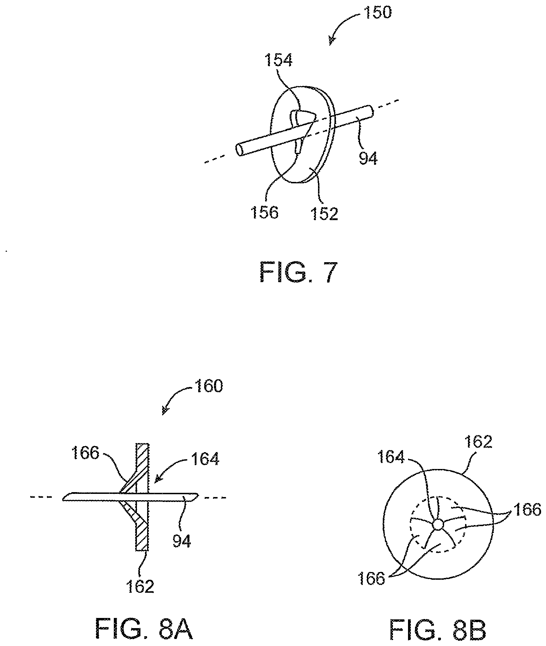

Fig. 7 shows a perspective view of another locking anchor variation **150** in which anchor body **152** defines an opening **154** having a tapered or grooved portion **156.** Opening **154** may be sized to allow suture **94** to pass through opening **154** such that anchor body **152** may be translated freely relative to suture **94.** Once anchor body **152** has been desirably positioned relative to the tissue fold or to the opposing anchor, suture **94** may be manipulated to slide into tapered or grooved portion **156,** which preferably defines a diameter which is less than a diameter of suture **94.** Sliding suture **94** into tapered or grooved portion **156** may lock a position of anchor body **152** relative to suture **94** due to the cleating effect of grooved portion **156** on suture **94.**

Figs. 8A and 8B show cross-sectional side and top views, respectively, of another locking anchor variation **160** in which anchor body **162** may define a through-hole passage or opening **164** through which suture **94** may pass. Anchor body **162** may have one or several levered, flapped, or biased locking members **166** which may be integrally formed with anchor body **162.** These locking members **166** may be formed radially about opening **164** such that when suture **94** is absent, the resting configuration of locking members **166** define an opening **164** having a diameter less than that of the suture **94** passed through. Locking members **166** may be biased to protrude in a single direction, as shown in Fig. 8A, such that when anchor body **162** is moved in a first direction over suture **94,** the anchor **162** passes freely. However, when anchor body **162** is moved in the opposing direction over suture **94,** locking members **166** engage onto suture **94** and prevent any reverse translation, thereby enabling uni-directional movement and locking of anchor body **162.** Although five locking members **166** are shown, any number of members may be utilized as practicable and as desired to effect a desired degree to locking.

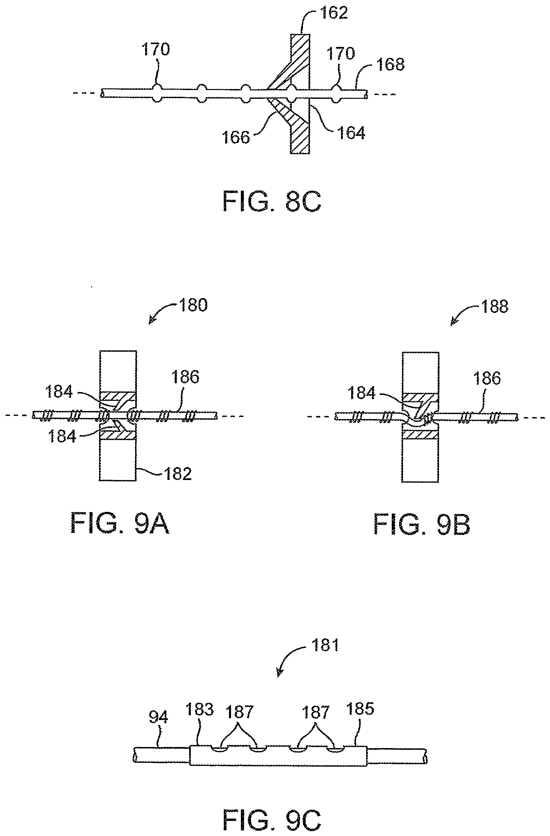

Fig. 8C shows a cross-sectional side view of anchor body **162** in combination with a modified suture **168** having integrated features or protrusions **170** defined along its length. Features or protrusions **170** may be defined uniformly at regular intervals along the length of suture **168** or intermittently, depending upon the desired effects, to enhance the locking ability of the anchor body onto the suture. Moreover, the features or protrusions **170** may be integrally formed protrusions or they may simply comprise knotted sections of suture. Sutures which are modified or knotted may be optionally utilized in any of the locking anchor variations as described herein in place of conventional sutures, depending upon the desired degree of locking and locking effects.

As shown in the cross-sectional views of Figs. 9A and 9B of locking anchor variations **180** and **188,** respectively, anchor body **182** may also comprise biased locking members **184,** contained within the anchor body **182.** The number and configuration of locking members **184** may be varied as desired and may optionally be apposed, as shown in Fig. 9A, or utilize a single member **184,** as shown in anchor variation **188** in Fig. 9B. The figures show knotted suture **186** used with anchor variation **180;** however, conventional sutures may also be utilized.

Fig. 9C shows another modification of suture **94** which may be utilized with any of the anchor locking variations shown herein. The portions of suture **94** which come into contact with the anchor locking mechanisms may be coated with a material having a relatively higher frictional coefficient, i.e., a coefficient of friction that is higher than the underlying suture material. For example, the portion of suture **94** may be coated with a metallic covering or slid within sleeve **181,** which may be made of a metallic material such as Titanium, Nitinol, stainless steel, etc. to enhance the locking force between suture **94** and the anchor. As shown in the figure, if sleeve **181** is utilized, the ends **183, 185** of sleeve **181** may be crimped onto suture **94.** One or several openings **187** may also be defined along sleeve **181** to further enhance the locking capability between suture **94** and the locking mechanism.

Aside from the use of mechanical locking features integrated within or with the anchor bodies, locking mechanisms may also utilize a variety of knotting techniques. Conventional knots, which are typically tied by the practitioner either within the body or outside the body and advanced over the suture length, may be utilized for locking the anchor in place relative to the tissue fold and opposing anchor; however, self-locking knots which enable the uni-directional travel of an anchor body relative to the suture and tissue are desirable.

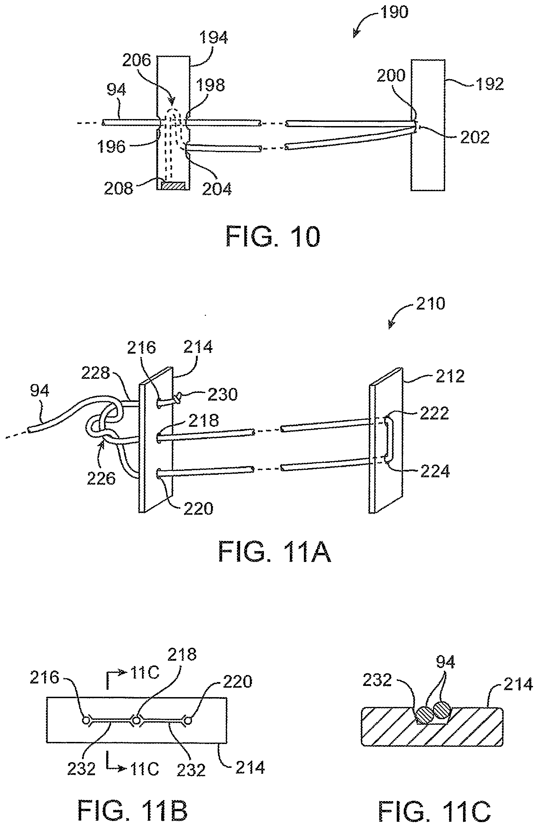

Fig. 10 shows locking anchor assembly **190** with distal anchor **192,** which may be positioned distally of a tissue fold, and proximal anchor **194,** which may be positioned proximally of a tissue fold or folds. In this variation, suture **94** may be routed through proximal anchor **194** via openings **196, 198** and extended to distal anchor **192.** At distal anchor **192,** suture **94** may be routed through opening **200** and over pin **202** positioned within distal anchor **192.** Pin **202** may function as a pulley over which suture **94** may travel during anchor locking adjustments. Suture **94** may then be routed back towards proximal anchor **194** through opening **204** and define loop **206** through which the proximal portion of suture **94** passes to thereby create a choke-type loop. The terminal end of suture **94** may then be anchored at fixed end **208** within the body of proximal anchor **194.**

In operation, when tension is applied to suture **94** or when proximal anchor **94** is advanced distally, proximal anchor **194** and distal anchor **192** may be freely drawn towards one another to secure any tissue fold or folds (not shown for clarity) disposed therebetween. However, if proximal anchor **194** were pulled or urged in the opposite direction away from the tissue or from distal anchor **192,** loop **206** would "choke" suture **94** and prevent any reverse movement of proximal anchor **194.**

Fig. 11A shows a perspective view of locking anchor assembly **210** having distal anchor **212** and proximal anchor **214** with suture **94** extending between the two anchors. Terminal end **230** of suture **94** may be knotted or otherwise retained by proximal anchor **214** and routed through opening **216** and back through opening **218** to create looped portion **228,** both openings **216, 218** being defined in proximal anchor **214.** Suture **94** may be routed from opening **218** and through distal anchor **212** via openings **222, 224.** Suture **94** may then be routed back to proximal anchor **214** through an opening **220** and wrapped **226** about looped portion **228** to continue on proximally. This knotted configuration facilitates advancement of proximal anchor **214** towards distal anchor **212** but chokes suture **94** when proximal anchor **214** is moved in an opposing direction.

Figs. 11B and 11C show top and cross-sectional side views of an alternative variation on proximal anchor **214** (and distal anchor **212,** if desired). As shown, anchor **214** may optionally define grooves or channels **232** which extend at least partially between openings **216, 218,** and **220.** These grooves or channels **232,** as seen in Fig. 11C, may be sized such that any of the overlying suture **94** are cinched or wedged into grooves **232** to facilitate the cinching action of anchor **214** with respect to suture **94.**

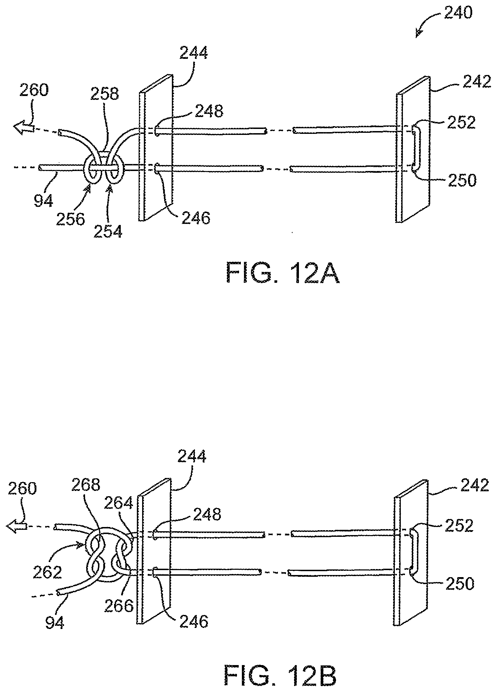

Another locking anchor assembly **240** is shown in the perspective view of Fig. 12A, which shows distal anchor **242** and proximal anchor **244** with suture **94** extending between the two anchors. Suture **94** may be routed through opening **246** defined through proximal anchor **244** and passed through distal anchor **242** via openings **250, 252.** Suture **94** may then be routed back towards proximal anchor **244** and passed through opening **248** to create at least two adjacent loops (half hitch knots) **254, 256** with looped section **258.** During cinching of proximal anchor **244** against the tissue, the knotted suture may be slid distally with proximal anchor **244.** Once proximal anchor **244** has been desirably positioned along suture **94,** the terminal end of suture **94** may be pulled, as shown by arrow **260,** to alter the knot configuration, commonly called changing the dressing of the knot, such that the knot becomes locked onto suture **94** and prevents any reverse movement of proximal anchor **244.**

Fig. 12B also shows a perspective view of another locking anchor variation similar to that shown in Fig. 12A. In this variation, suture **94** may be wrapped into two intertwined loops **264, 266** and further wrapped again into adjacent intertwined loops **262, 268.** Distal advancement of the knotted configuration along with proximal anchor **244** may be accomplished until the terminal end of suture **94** is placed under tension, as shown by arrow **260.** Tension may be applied once proximal anchor **244** has been desirably positioned along suture **94** to lock the knot into position and prevent any reverse movement of proximal anchor **244** along suture **94.**

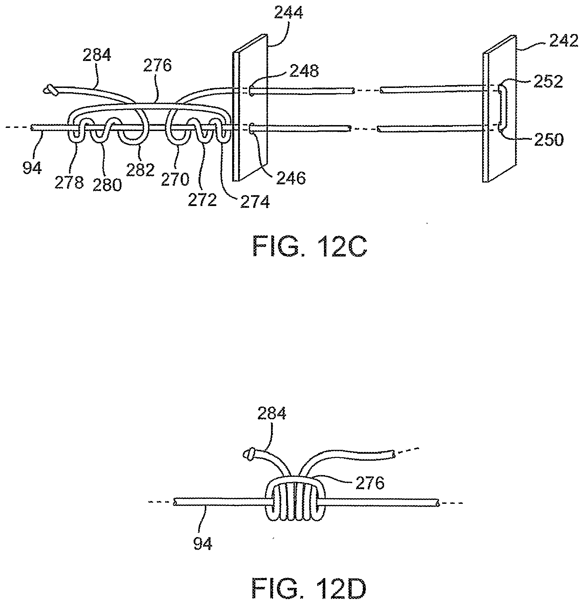

Fig. 12C shows a perspective view of another anchor locking assembly similar to the variations above. The knot may be modified by wrapping suture **94** into a first set of several loops, shown as three loops **270, 272, 274,** although in other variations, any number of loops may be utilized depending upon the desired locking effects. Suture **94** may then be wrapped in a second set of several additional loops in a proximally adjacent position about suture **94,** shown as loops **278, 280, 282** joined by looped section **276.** Likewise, any number of loops in the second set may be utilized either independent of the number of loops in the first set or to mirror the first set of loops. In either situation, once suture terminal end **284** is tightened, a knotted configuration, as shown in Fig. 12D, is formed which may be freely slid along suture **94** provided the knotted configuration itself is pushed along suture **94,** e.g., via a pusher tube, knot pusher, etc. However, once tension is applied along suture **94** by proximal anchor **244** pushing against the knot and by the tension created in the suture extending between anchors **242, 244,** the knot locks against suture **94** and prevents reverse movement of proximal anchor **244** along suture **94.**

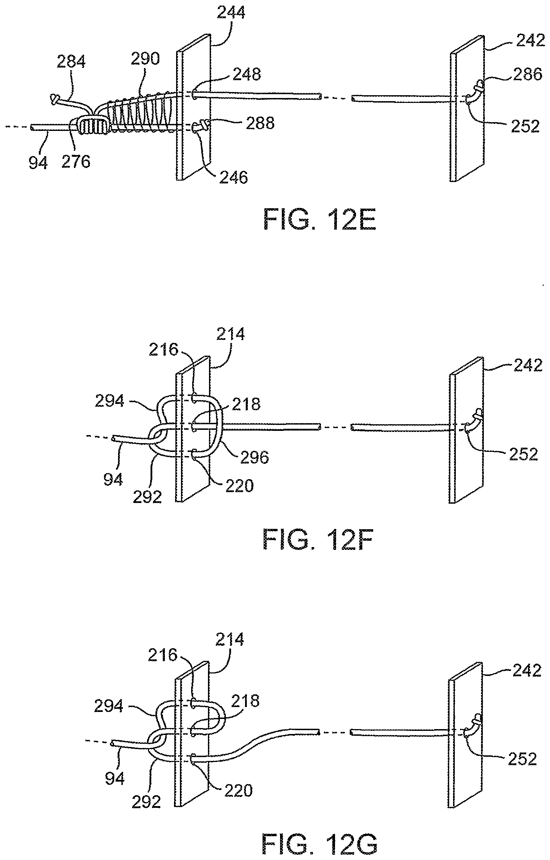

Fig. 12E shows a perspective view of another locking anchor variation similar to the variation shown in Fig. 12D yet having a single suture traverse between anchors **242, 244.** In this variation, suture **94** may have terminal end **286** anchored or retained by distal anchor **242** at opening **252** and have a single suture traverse to proximal anchor **244.** A second terminal end **288** may also be anchored or retained by proximal anchor **244** at opening **246.** The portions of suture **94** extending between proximal anchor **244** and the knot may have a biasing member, e.g., spring **290,** disposed over one or both lengths of suture to maintain proximal anchor **244** and the knot under a constant force to ensure that the knot is maintained under a locking force to prevent the reverse travel of proximal anchor **244.**

Yet another variation of a locking anchor variation having a single suture traversing the anchors is shown in the perspective view of Fig. 12F. A terminal end **252** of suture **94** may be anchored or retained at distal anchor **242** and routed to proximal anchor **214** through opening **218.** The length of suture **94** may form loop **292** on a first side of proximal anchor **214** and a second loop **296** on the opposite side of proximal anchor **214** between openings **216, 220.** Suture **94** may then be wrapped about loop **292** via loop **294** on the first side to form an interlocking suture loop. This variation is also effective in allowing proximal anchor **214** to translate over suture **94** towards the tissue and distal anchor **242** yet prevent reverse movement of proximal anchor **214** due to a choking action by the intertwined suture loops on the proximal side of proximal anchor **214.**

Fig. 12G shows a perspective view of another locking anchor variation similar to that shown in Fig. 12F. Here, suture **94** may be routed through opening **220** in proximal anchor **214** to form loop **292** before being passed through openings **218** and **216** and intertwining loop **294** through loop **292.** Likewise, this variation is also effective in allowing proximal anchor **214** to translate over suture **94** towards the tissue and distal anchor **242** yet prevent reverse movement of proximal anchor **214.**

As mentioned above, the locking and cinching mechanisms described herein may be utilized with a variety of different anchor types. For instance, the cinching mechanisms described above may be used not only with T-type anchors but also with reconfigurable basket-type anchors. Described hereinafter are basket-type anchors configured for implantation or placement against tissue in a similar manner as described previously and examples of how cinching mechanisms may be utilized in securing tissue plications. Moreover, additional cinching mechanisms which are preferably utilizable with basket-type anchors are also described below.

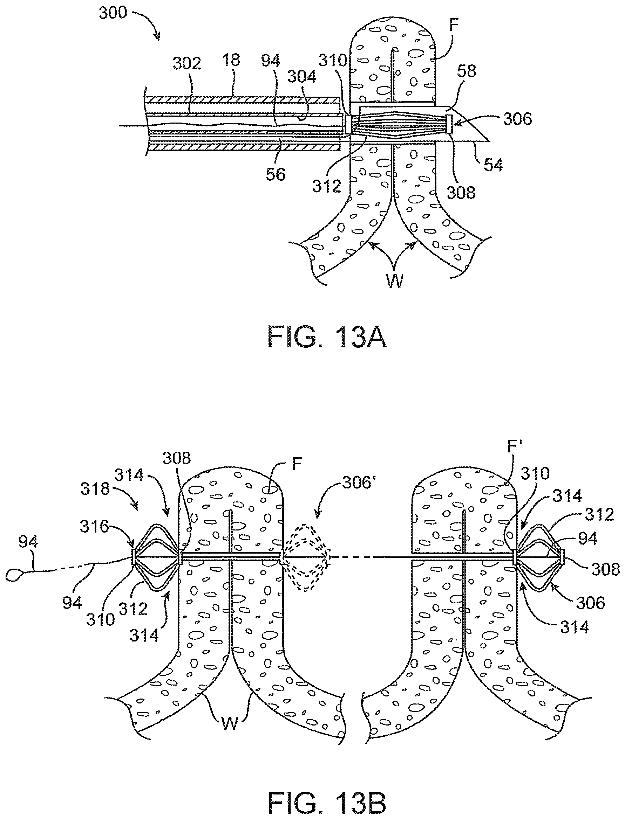

When cinching or locking basket-type anchors, the baskets may be delivered into or through the tissue in the same or similar manner as described above, particularly as shown in Figs. 3A-3G. For example, Fig. 13A shows anchor delivery system 300 in proximity to tissue fold **F.** Again, tissue fold **F** may be disposed within a gastrointestinal lumen, such as the stomach, where tissue wall **W** may define the outer or serosal layer of the stomach. Delivery push tube or catheter **302** may be disposed within launch tube **18** proximally of basket anchor **306,** which is shown in a compressed delivery configuration with a relatively low profile when disposed within needle lumen **58** of needle **54.** A single basket anchor **306** is shown disposed within needle **54** only for illustrative purposes and is not intended to be limited by the number of basket anchors; rather, any number of basket anchors may be disposed within needle lumen **58** as practicable depending upon the desired procedure and anchoring results.

Suture **94** may be routed through or externally of push tube lumen **304** and further routed within and/or through proximal collar **310** of anchor **306.** The terminal end of suture **94** may be routed within anchor **306** and affixed to distal collar **308** in one variation. Alternatively, suture **94** may be affixed or anchored within anchor **306** or at proximal collar **310** depending upon the desired effect and procedure being performed. Moreover, if multiple anchors are utilized in a tissue plication procedure, suture **94** may be routed through anchor **306** such that the anchor **306** may freely slide along or over suture **94.**

The basket anchors may comprise various configurations suitable for implantation within a body lumen. Basket anchors are preferably reconfigurable from a low profile delivery configuration to a radially expanded deployment configuration in which a number of struts, arms, or mesh elements may radially extend once released from launch tube 18 or needle 54. Materials having shape memory or superelastic characteristics or which are biased to reconfigure when unconstrained are preferably used, e.g., spring stainless steels, Ni-Ti alloys such as Nitinol, etc. The basket anchor 306 is illustrated as having a number of reconfigurable struts or arm members 312 extending between distal collar 306 and proximal collar 310; however, this is intended only to be illustrative and suitable basket anchors are not intended to be limited to baskets only having struts or arms. Examples of suitable anchors are further described in detail in U.S. Pat. App. Serial No. 10/612,170 (US Publication No. 2004-0122456).

Fig. 13A shows basket anchor 306 delivered through tissue fold F via needle 54 and launch tube 18. As above, the other parts of the plication assembly, such as upper and lower bail members 20, 26, respectively, and tissue acquisition member 28 have been omitted from these figures only for clarity.

Fig. 13 B shows one variation where a single fold F may be secured using basket anchor 306'. As seen, basket anchor 306' has been urged or ejected from needle 54 and is shown in its radially expanded profile for placement against the tissue surface. In such a case, a terminal end of suture 94 may be anchored within the distal collar of anchor 306' and routed through tissue fold F and through, or at least partially through, proximal anchor 318, where suture 94 may be cinched or locked proximally of, within, or at proximal anchor 318 via any number of cinching mechanisms 316 described herein. Proximal anchor 318 is also shown in a radially expanded profile contacting tissue fold F along tissue contact region 314. Locking or cinching of suture 94 proximally of proximal anchor 318 enables the adequate securement of tissue fold F.

If additional tissue folds are plicated for securement, distal basket anchor 306 may be disposed distally of at least one additional tissue fold F', as shown in Fig. 13B, while proximal anchor 318 may be disposed proximally of tissue fold F. As above, suture 94 may be similarly affixed within distal anchor 306 and routed through proximal anchor 318, where suture 94 may be cinched or locked via proximal anchor 318, as necessary. If tissue folds F and F' are to be positioned into apposition with one another, distal basket anchor 306 and proximal anchor 318 may be approximated towards one another. As described above, proximal anchor 318 is preferably configured to allow suture 94 to pass freely therethrough during the anchor approximation. However, proximal anchor **318** is also preferably configured to prevent or inhibit the reverse translation of suture **94** through proximal anchor **318** by enabling uni-directional travel of anchor **318** over suture **94.** This cinching feature thereby allows for the automated locking of anchors **306, 318** relative to one another during anchor approximation.

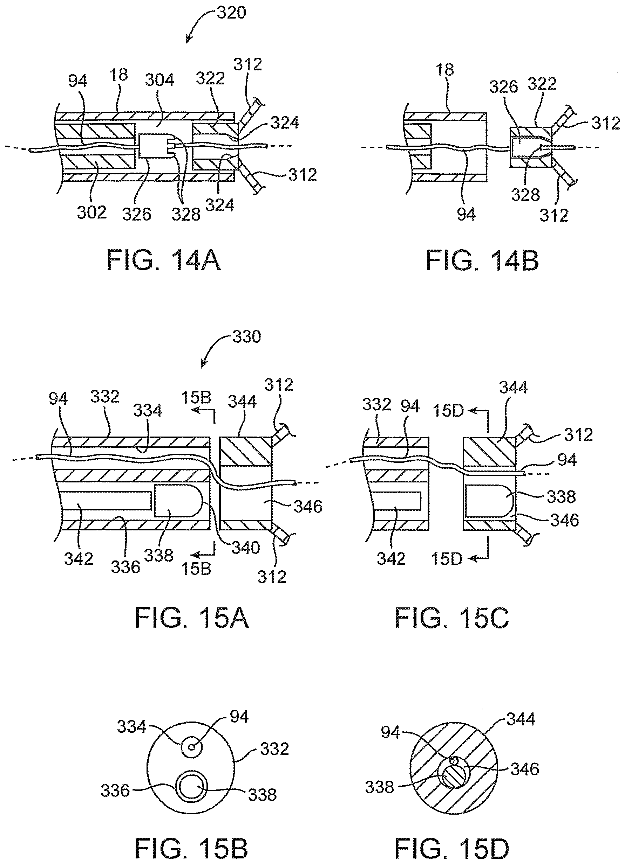

Aside from the anchor cinching or locking mechanisms utilizing looped and knotted sutures for facilitating uni-directional locking, various mechanisms utilizing friction may also be implemented. Figs. 14A and 14B show cross-sectional side views of one variation in cinching assembly **320.** Proximal collar **322,** proximal portions of struts **312,** and distal portions of launch tube **18** are shown and other features of the assembly and tissue fold **F** have been omitted from the figure only for clarity.

A locking or cinching collar or collet **326** may be positioned within launch tube **18** proximally of anchor collar **322.** Cinching collet **326** may comprise a cylindrically shaped member defining a lumen therethrough for passage of suture **94.** A distal end of cinching collet **326** may have at least one and preferably several clamping arms or teeth **328** which are configured to cinch or clamp down upon suture **94** passing through. Proximal anchor collar **322** may be sized to correspondingly receive cinching collet **326** therewithin to create an interference fit. relative to an outer diameter of cinching collet **326.** A distal portion of anchor collar **322** may also define a tapered or angled portion **324** such that when cinching collet **326** is advanced within anchor collar **322,** angled portion **324** may effectively force clamping arms or teeth **328** to cinch radially inward upon suture **94.**

In operation, once proximal anchor **318** has been desirably positioned relative to tissue fold **F** and/or the distal anchor and with proximal collar **322** positioned within launch tube **18,** delivery push tube **302** may be advanced distally to urge cinching collet **326** into anchor collar **322** such that clamping arms or teeth **328** are clamped onto suture **94** and cinching collet **326** is friction-fitted within anchor collar **322.** Anchor collar **322** may then be urged out of launch tube **18** and the anchor left against the tissue surface.

Another cinching assembly variation **330** is shown in the cross-section view of Figs. 15A and 15C. Launch tube **18** has been omitted from these figures for clarity only. Delivery push tube **332** is shown as defining suture lumen **334** and locking member or pin lumen **336** therethrough. Although two separate lumens are shown, a single common lumen may be utilized in alternative variations. With proximal anchor collar **344** positioned distally of push tube **332,** suture **94** may be routed through suture lumen **334** and through collar lumen **346.** Locking member or pin **338** may be positioned within lumen **336** proximally of collar lumen **326.** Fig. 15B shows an end view of push tube **332** with locking pin **338** and suture **94** positioned within prior to cinching of the anchor.

Once the anchor has been desirably positioned relative to the tissue, suture **94** may be pulled proximally such that anchor collar **344** rests against the distal end of push tube **332.** Locking pin **338,** which may define a tapered or radiused end **340** to facilitate its insertion into collar lumen **346,** may be urged distally via push rod **342** to force locking pin **338** into anchor collar **344** such that the portion of suture **94** within anchor collar **344** becomes effectively wedged and thereby prevents further movement of the anchor along suture **94.** Fig. 15C shows a cross-sectional side view of locking pin **388** having been urged into anchor collar **344** in a frictional engagement with suture **94.** Fig. 15D shows a cross-sectional end view of collar **344** with locking pin **388** and suture **94** positioned within.

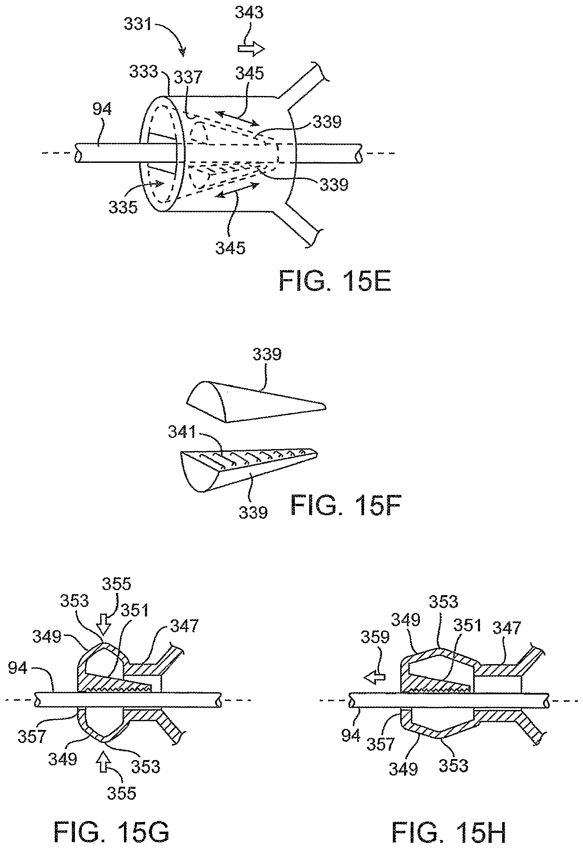

Fig. 15E shows a perspective view of another cinching variation **331** which is similar to the variation described above. One or more tapered pins or blocks **339** may be slidably disposed within tapered channel **335** defined in proximal collar **333.** The figure shows two tapered pins **339,** although a single pin may be utilized or more than two pins may also be used. If two or more pins **339** are utilized, suture **94** may be passed between the pins **339.** Pins **339** may be free to slide along inner surface **337** of channel **335** in the direction of arrows **345** depending upon the direction of travel of suture **94** through channel **335.** Fig. 15F shows a perspective view of only pins **339** for clarity; as seen, pins **339** may be tapered distally from a larger diameter to a smaller diameter and although pins **339** are shown as semi-circularly shaped members, contact surface **341** may be curved or arcuate to better contact suture **94.** Moreover, contact surface **341,** which contacts suture **94** passing through channel **335,** may define a roughened surface or it may alternatively define a plurality of serrations, teeth, projections, etc., to facilitate contact against suture **94.**

In use, as proximal collar **333** is translated in the direction of arrow **343,** pins **339** may be forced proximally such that suture **94** may pass freely through channel **335.** However, if proximal collar **333** were to be translated in the opposing direction, pins **339** may be forced in the opposite direction to cinch down upon suture **94** within channel **335** and thereby inhibit any further motion.

An alternative variation of the assembly is shown in the cross-sectional views of Figs. 15G and 15H, which show a cinching anchor having a retractable pin. Fig. 15G shows proximal collar **347** with one or more retracting arms **349** extending proximally from collar **347.** Retracting arms **349** may be configured to pivot at bend **353** when urged via a compression force applied at bend **353** in the direction of arrows **355.** The application of this compression force may urge pin support collar **357** which is defined at a proximal portion of arms **349,** to move in the direction of arrow **359.** This in turn may move pin **351,** which extends from pin support collar **357,** proximally out of proximal collar **347** to thereby release suture **94** from its locked position. In one variation, retracting arms **349** may be biased to retain pin **351** within proximal collar **347** unless a compression force is applied at bend **353.**

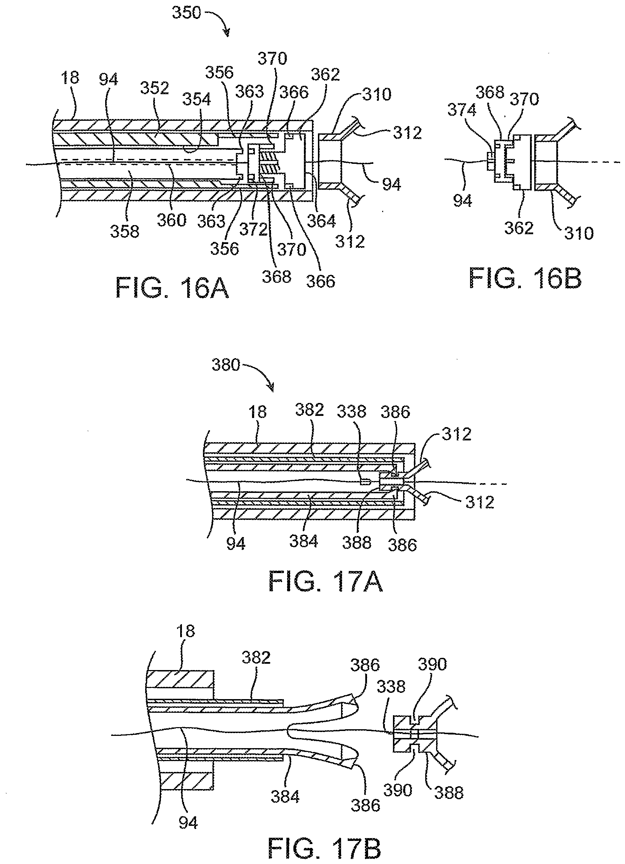

Figs. 16A and 16B show cross-sectional side views of another variation of cinching assembly **350.** Cinching assembly **350** may generally comprise outer tubing **352** and inner tubing **358** rotatingly positioned within outer tubing lumen **354.** Cinching member **362** may be positioned distally of outer tubing **352** and may generally comprise a collar base **364** and cinching collar **374** projecting proximally from collar base **364.** Cinching collar **374** is preferably tapered and threaded and may also be longitudinally slotted such that rotatable collar **368** may be rotatingly disposed upon slotted cinching collar **374.** A distal end of outer tubing **352** may define one or several engaging members **356** which are adapted to engagingly contact detents or keyed engagement interfaces **366** located on collar base **364.** Inner tubing **358** may also define one or several engaging members **363** which are also adapted to engagingly contact detents or keyed engagement interfaces **372** located on the rotatable cinching collar **374.** Suture **94** may be routed through inner tubing lumen 360, through cinching collar **374,** and through proximal anchor collar **310.**

In operation, suture **94** may pass freely through assembly **350.** Once the anchor has been desirably positioned, engaging members **356** on outer tubing **352** may be correspondingly engaged against interface **366** and engaging members **363** on inner tubing **358** may be engaged against interface **372.** With suture **94** tensioned appropriately, outer tubing **352** may be held stationary while inner tubing **358** is rotated to torque rotatable collar **368** about threaded cinching collar **374.** As rotatable collar **368** is torqued onto cinching collar **374,** the tapered shape may urge the slotted members to cinch upon suture **94** passing therethrough. Stand-offs **370,** which may protrude from rotatable collar **368,** may be adjusted in height to control how far rotatable collar **368** may be torqued onto collar base **364** such that the degree to which rotatable collar **368** is torqued about cinching collar **374** may be desirably adjusted. Once the cinching collar **374** has been desirably cinched onto suture **94,** proximal anchor collar **310** may be ejected from launch tube **18** along with the cinching assembly, as shown in Fig. 16B.

Another variation on cinching assembly **380** may be seen in the cross-sectional views of Figs. 17A and 17B. Assembly **380** is similar to cinching assembly **330** shown above in Figs. 15A to 15D. Delivery push tube **332** and push rod **342** have been omitted from these figures only for clarity. In this variation, when locking pin **338** is pushed distally into proximal collar **388,** a retaining tube member **384** may be utilized to provide a counterforce to stabilize proximal collar **388** during cinching. Retaining tube member **384** may generally comprise one or several collar engaging arms **386** for engaging proximal anchor collar **388** at collar detents **390** defined along anchor collar **388.** During the insertion of locking pin **338** into collar **388,** collar engaging arms **386** may be positioned within launch tube **18** or within retractable sleeve **382.** After locking pin **338** has been inserted within anchor collar **388,** engaging arms **386** may be advanced distally out of launch tube **18** or retractable sleeve **382** may be withdrawn proximally to expose engaging arms **386.** Once free of any constraining forces, engaging arms **386** may be biased to spring or open radially to then release proximal anchor collar **388** in a cinched configuration.

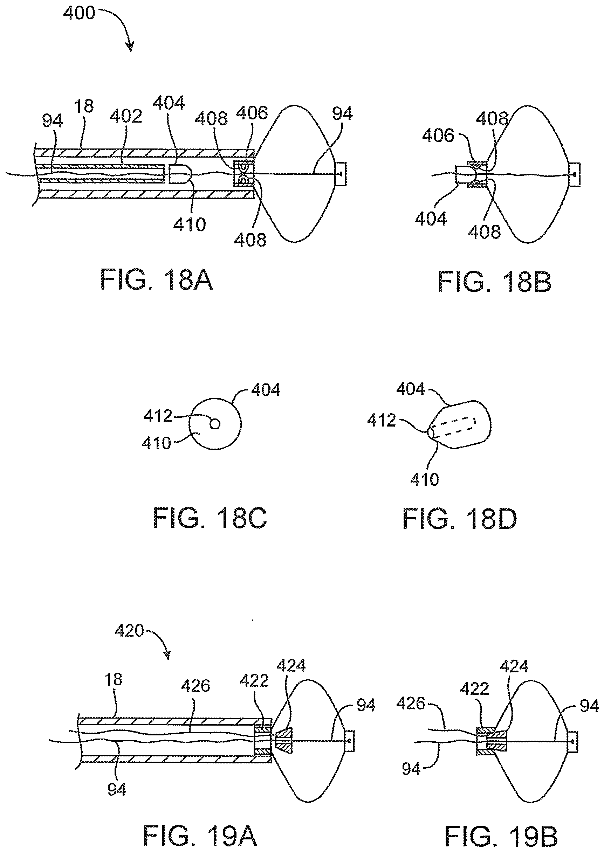

Another variation on cinching assembly **400** is shown in Figs. 18A to 18D, in which proximal anchor collar **406** may comprise one or several biasing members or cinching tabs **408** within collar **406.** Each of the tabs **406** may be biased to project inwardly such that suture **94** passing through is automatically cinched and locked in position, as shown in Fig. 18A. A suture release member **404,** which may generally comprise a cylindrically shaped tube or member having a tapered surface **410** and a suture lumen **412** defined therethrough, may be positioned within anchor collar **406** during anchor positioning to allow free passage of suture **94** through the anchor, as shown in Fig. 18B. Fig. 18C shows an end view of release member **404** defining suture lumen **412** extending therethrough. Fig. 18D shows a perspective view of release member **404** to show tapered surface **410** and suture lumen **412** in better detail. Tapered surface **410** may be omitted but is preferably to facilitate the insertion and removal of release member **404** from anchor collar **406.** When the anchor has been desirably positioned and is ready to be locked in place over suture **94,** tubular member **402** may engage suture release member **404** for withdrawal from anchor collar **406.** The removal of release member **404** may cause cinching tabs **408** to lock upon suture **94** and prevent the further movement of the anchor relative to suture **94.**

Figs. 19A and 19B show another variation of cinching assembly **420** in which a deformable cinching member **424** may be positioned within the anchor distally of anchor collar **422.** Cinching member **424** may define a tapered outer surface such that when the anchor is ready to be secured to suture **94,** the insertion of cinching member **424** into collar **422** may compress cinching member **424** about suture **94** such that any further movement of the anchor is prevented. Cinching member **424** may be pulled into anchor collar **422** via pull wire **426,** which may be manipulated at its proximal end by the surgeon or user when desired.

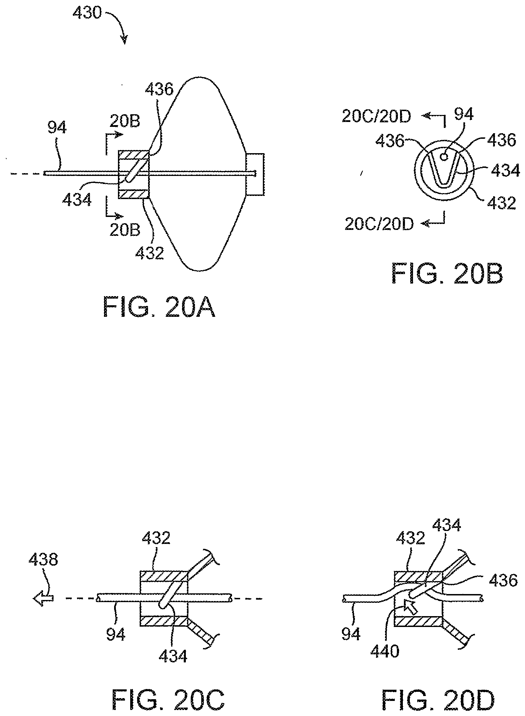

Figs. 20A to 20D show cross-sectional views of another variation in cinching assembly **430.** As shown in Fig. 20A, proximal anchor collar **432** may comprise a pivotable locking member **434** contained either within anchor collar **432** or proximally of collar **432.** This example illustrates locking member **434** contained within collar **432.** Suture **94** may pass through locking member **434,** which is shown in the end view of collar **432** in Fig. 20B, as having two pivots **436.** Moreover, locking member **434** and pivots **436** may be integrally formed from proximal collar **432.** Pivoting locking member **434** may be biased to rotate about pivot **436** such that a resting position of locking member **434** is against an inner surface of collar **432.** During distal anchor translation over suture **94,** tension as represented by arrow **438,** on suture **94** may force pivot **434** into an open position where suture **94** may pass freely through. Upon having desirably positioned the anchor against tissue, locking member **436** may be biased to pivot in direction **440** to lock suture **94** against the inner surface of collar **432.** Opposite movement of the anchor relative to suture **94** may act to further cinch locking member **434** against suture **94** and thereby further inhibit the movement of the anchor in the reverse direction.

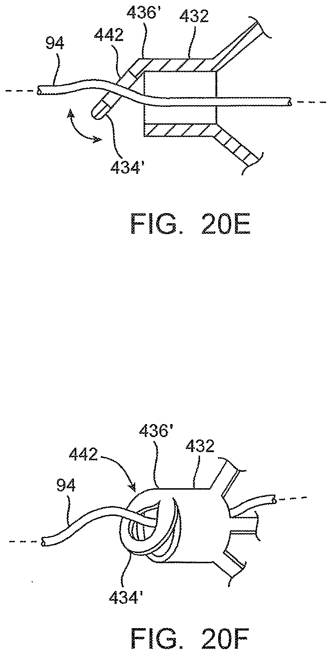

A similar variation is shown in the cross-sectional side and perspective views of Figs. 20E and 20F, respectively. In this variation, locking member **434'** may extend proximally of proximal collar **432** at an angle relative to collar **432.** Locking member **434'** may be pivotable via pivot **436'** such that locking member **434'** may pivot in the direction of the arrows shown depending upon the direction which the tissue anchor is translated relative to suture **94.** If proximal collar **432** is translated distally over suture **94,** it may travel freely; however, if proximal collar **432** is translated proximally in the opposite direction, suture **94** may become wedged in a tapered portion of opening **442** through which suture **94** passes. Once suture **94** is wedged in tapered opening **442,** locking member **434'** may pivot towards proximal collar **432,** where it is stopped from further motion, thereby locking the tissue anchor onto suture **94** and preventing its reverse motion.

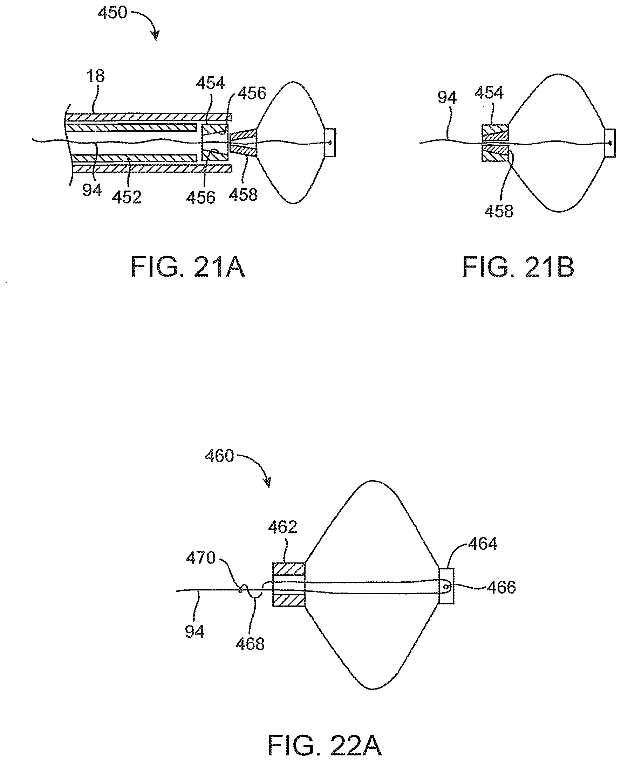

Figs. 21A and 21B show another cinching assembly variation **450** as seen in the cross-sectional side views. In this variation, delivery push tube **452** may be disposed proximally of locking collar **454** and proximal tapered anchor collar **458.** Once the anchor has been desirably positioned relative to the tissue fold **F,** with suture **94** under tension, locking collar **454** may be urged distally via push tube **452** such that locking collar **454** slides over proximal anchor collar **458.** An inner surface of locking collar **454** may be tapered and anchor collar **458** may also be tapered in a correspondingly opposed manner such that when locking collar **454** is mated with anchor collar **458,** anchor collar **458** may cinch locking collar **454** upon suture **94** to thereby prevent any further movement of the anchor over suture **94.** Both collars may be made from any of the same or similar materials, as described above.

In addition to friction-based locking and cinching mechanisms utilizable in tissue anchors, other mechanisms which create tortuous paths for the suture within or through the anchors may also be utilized for creating uni-directional locking.

One cinching anchor variation **460** is shown in the cross-sectional side view in Fig. 22A. As shown, suture **94** may be routed through anchor proximal collar **462** and looped over pulley or pin **466** contained within distal collar **464.** Suture **94** may then be routed back through and looped **468** about suture **94** and tied with slip knot **470.** As tension is applied to suture **94,** slip knot **470** may prevent further movement of the anchor relative to suture **94.**

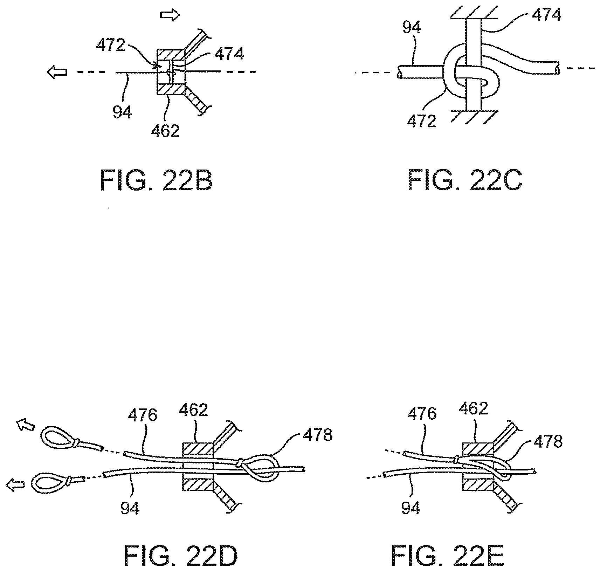

Figs. 22B and 22C show a variation which may be used in combination with cinching anchor variation **460** or alone. Pin **474** may optionally be positioned within proximal collar **462** and suture **94** may be wrapped or looped about itself around pin **474** in a manner as shown in the detail view of Fig. 22C. The configuration of loop **472** may allow for the uninhibited translation of the anchor in the direction of the arrow as shown; however, when the anchor is moved in the opposite direction, loop **472** may effectively cinch upon itself to thus prevent or inhibit the reverse motion of the anchor relative to suture **94.**

Another cinching variation is shown Figs. 22D and 22E. Suture **94** may be routed through proximal collar **462** with an additional length of cinching suture **476.** The distal end of cinching suture **476** may form loop **478** which is wrapped about suture **94** and free to slide over suture **94.** After the anchor has been desirably positioned relative to the tissue and with suture **94** preferably under tension, cinching suture **476** may be pulled proximally such that loop **478** is pulled into proximal collar **462** and becomes wedged against suture **94.** The multiple lengths of suturing material utilized for loop **478** and suture **94** preferably form a cross-sectional area which is larger than an inner diameter of proximal collar **462** such that positioning loop **478** and suture **94** within collar **462** ensures a frictional lock which prevents further movement of the suture **94** relative to the anchor. Suture **94** and cinching suture **476** are preferably made from the same or similar materials although differing suture materials may also be used.

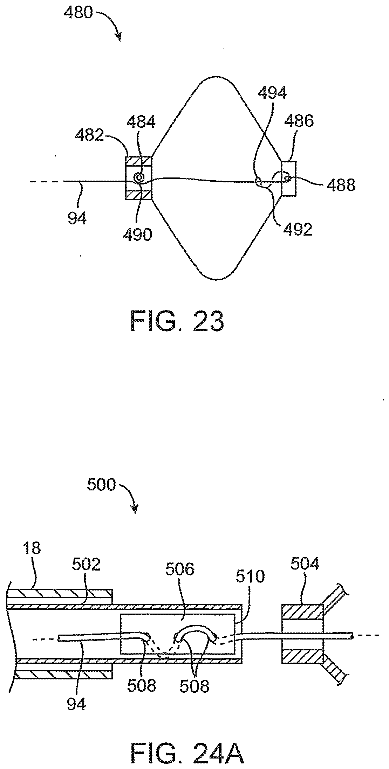

Fig. 23 shows a cross-sectional view of cinching assembly variation **480** in which proximal collar **482** may comprise pulley or pin **484** about which suture **94** may be looped once or several times **490.** Distal collar **486** may also comprise pulley or pin **488** about which suture **94** may also be looped once or several times before being wrapped or looped **492** back about suture **94.** The terminal end of loop **492** may be secured about suture **94** via slip knot **494.** This configuration of looping allows for the anchor to be advanced uni-directionally relative to the suture and tissue, yet prevents or inhibits the reverse movement of the anchor and thus effectively enables the tissue to be cinched via the anchors.

Another cinching or locking anchor variation **500** is shown in the cross-sectional view of Fig. 24A. In creating a tortuous path for suture **94,** cinching sleeve **506** may be positioned proximally of proximal collar **504** within or distally of delivery push tube **502.** Cinching sleeve **506** may generally comprise a tubular structure having sleeve lumen **510** defined therethrough and a number of openings **508** defined along the length of sleeve **506.** Openings **508** may be uniformly patterned along sleeve **506** or they may be randomly positioned. Moreover, any number of openings **508** may be utilized as practicable. In either case, suture **94** may be routed in various patterns throughout openings **508** and through sleeve lumen **510** before being routed through proximal collar **504.** Once the anchor is to be locked, cinching sleeve **506** may be urged distally via delivery push tube **502.** When urged or pushed distally, this may be done slowly so as to allow suture **94** to pass through the tortuous path created by suture **94** passing through openings **508.** However, once cinching sleeve **506** has been advanced proximally adjacent to proximal collar **504,** cinching sleeve **506** may become locked with proximal collar **504** pressing against sleeve **506.**

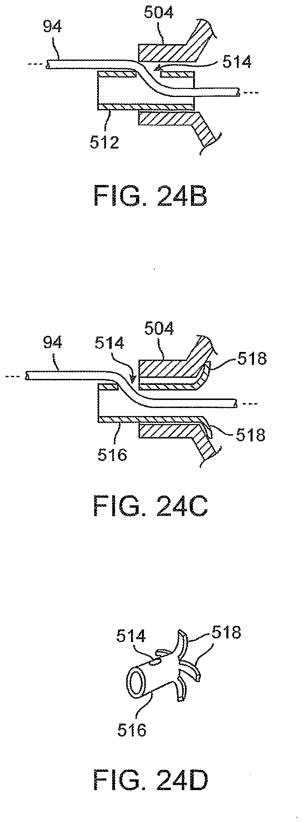

Fig. 24B shows another variation of a cinching mechanism which utilizes a tortuous path. Cinching sleeve **512** may comprise a tubular structure having an opening **514** defined along a surface of sleeve **512** through which suture **94** may pass. Cinching sleeve **512** may be disposed within proximal collar **504** with suture **94** routed from outside of sleeve **512** and passing to within sleeve **512** through opening **514.** In operation, because of the manner in which suture **94** is routed through sleeve **512** and into the anchor, distal translation of the anchor relative to the tissue and suture **94** is uninhibited. But when the anchor is reversed in direction relative to suture **94,** suture **94** and cinching sleeve **512** may be drawn into the anchor and become locked due to the interference between suture **94,** cinching sleeve **512,** and proximal collar **504.** Accordingly, an outer diameter of cinching sleeve **512** is preferably sized to be slightly less than an inner diameter of proximal collar **504** such that when suture **94** is passed through opening **514,** cinching sleeve **512** becomes wedged against collar **504.** Cinching sleeve **512** may be made from any of the same or similar materials as the anchors, as described above.

Figs. 24C and 24D show another variation similar to cinching sleeve **512** described above. Cinching sleeve variation **516** may be similarly sized as sleeve **512** and may also similarly define an opening **514;** however, sleeve **516** includes one or several retaining arms **518** defined on a distal end of sleeve **516.** Any number of retaining arms **518** may be utilized provided that they extend radially and reside distally of proximal collar **504** such that they prevent sleeve **516** from sliding proximally out of collar **504.** Fig. 24D shows a perspective view of cinching sleeve **516** with retaining arms **518** radially extended from the body of sleeve **516.** Cinching sleeve **516** may also be made from the same or similar material as the anchor; for example, sleeve **516** may be fabricated from a material having superelastic characteristics, such as Nitinol. Accordingly, when cinching sleeve **516** is initially inserted through collar **504** and/or during anchor delivery through launch tube **18** into or through the tissue, retaining arm or arms **518** may be configured into a low profile with arm or arms **518** constrained into a tubular shape. Upon anchor release or upon being inserted through collar **504,** retaining arms **518** may be released to extend radially.

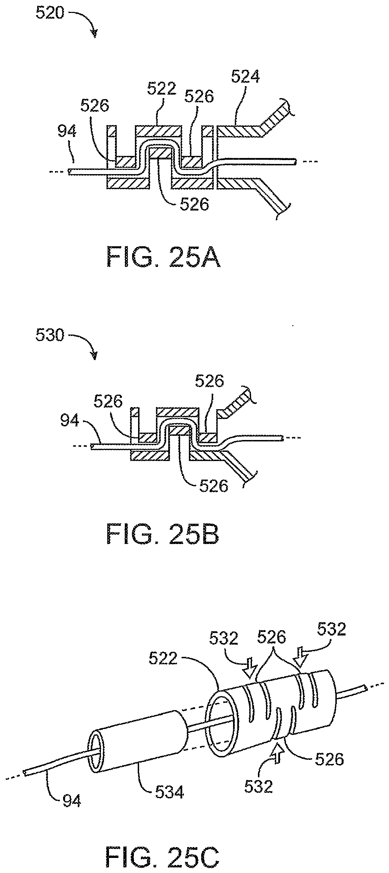

Yet another variation **520** on cinching assembly is shown in Fig. 25A. Generally, assembly variation **520** may comprise cinching collar **522** located proximally of anchor proximal collar **524.** Cinching collar **522** may be a tubular structure having superelastic material characteristics, such as those found in Nitinol. Obstructing members **526,** which may be formed from portions of cinching collar **522,** may be pressed or formed to extend into a lumen of cinching collar **522** such that a tortuous path is created for the passage of suture **94.** Although three obstructing members **526** are shown in the figure, any number of obstructions as practicable may be created depending upon the desired tortuous path to be created.

Assembly **520** shows cinching collar **522** as a separate collar located proximally of anchor collar **524;** however, the cinching collar may be integrated with the anchor collar such that a singular integral structure is formed, as shown in anchor variation **530** in the cross-sectional view of Fig. 25B. In either alternative during anchor placement relative to the tissue fold, retaining sleeve **534** may be inserted within cinching collar **522** to maintain obstructing members **526** in an open position for allowing suture **94** to pass freely through sleeve **534.** Once the anchor has been desirably positioned, retaining sleeve **534** may be withdrawn, as shown in the perspective view of Fig. 25C, using any number of methods. Removal of retaining sleeve **534** will allow for obstructing members **526** to reconfigure inwardly in the direction of arrows **532** to thus reconfigure cinching collar **522** into a tortuous path.

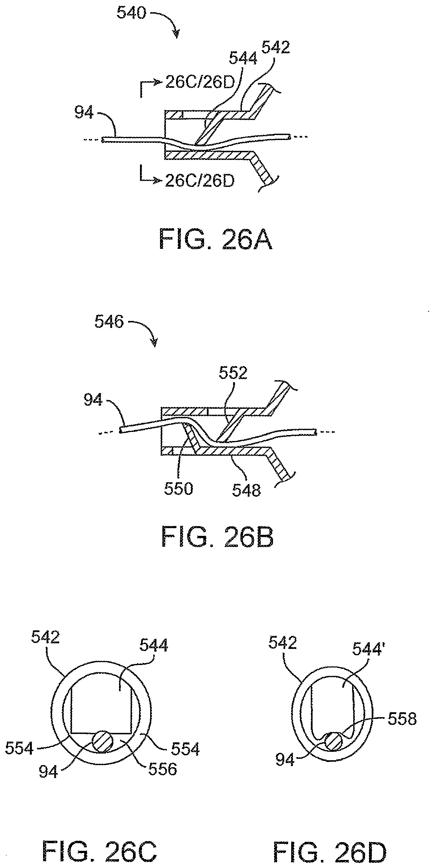

Cinching assembly **540** may also utilize a single or any number of tabs or levers to aid in capturing suture **94** and/or creating a tortuous path for suture **94** to traverse. As shown in the cross-sectional view of Fig. 26A, proximal collar **542** may have a pivoting lever **544** formed integrally from a side wall of proximal collar **542.** Alternatively, lever **544** may be included in a cinching collar separate from proximal collar **542.** Lever **544** may be biased to spring inwardly into proximal collar **542** upon suture **94** passing therethrough. During translation of the anchor in a first direction, suture **94** may be allowed to freely pass through proximal collar **542** and past lever **544** due to its pivoting motion. When the anchor is moved or urged in the reverse direction, lever **544** may act to cinch down upon suture **54** against an inner surface of proximal collar **542,** as shown in the figure.

An embodiment **546** according to the invention is shown in the cross-sectional view of Fig. 26B in which proximal collar **548** is shown as having at least two levers **550, 552** both biased in opposing directions to create a tortuous path for suture **94** to traverse. The cinching levers are configured to prevent or inhibit the over-cinching or cutting of suture **94.** Figs. 26C and 26D show alternative end views of Fig. 26A. Uni-directional lever **544,** as seen in Fig. 26C, may be formed from the side wall of proximal collar **542** such that when lever **544** cinches down upon suture **94,** the corners or ends of lever **544** contact an inner surface of proximal collar **542** at contact points **554.** The contact which occurs may ensure that an open space **556** is preserved and that lever **544** is prevented from over cinching onto suture **94** within space **556** and cutting suture **94.** Fig. 26D shows an alternative uni-directional lever **544'** which defines a curved or arcuate edge **558** which contacts suture **94.** The arcuate edge **558** may prevent the over cinching onto suture 94 and cutting of suture **94.**

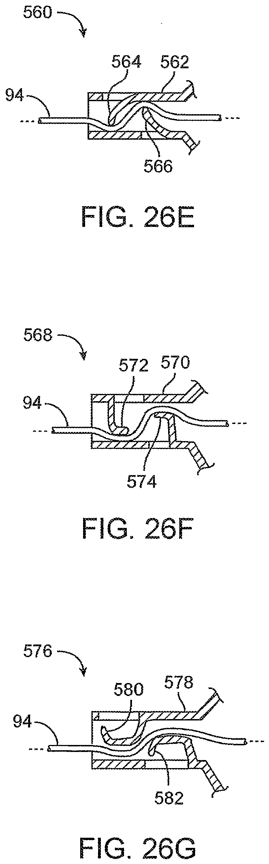

Figs. 26E, 26F, and 26G show alternative variations of cinching assembly **546** according to the invention with uni-directional levers having various configurations. Fig. 26E shows a cross-sectional side view of cinching assembly **560** in which proximal collar **562** has levers **564, 566** directed and biased in opposing directions to create a tortuous path. Each of the levers **564, 566** in this variation are curved inwardly towards proximal collar **562.** Fig. 26F shows a cross-sectional side view of cinching assembly **568** in which proximal collar **570** has uni-directional levers **572, 574** angled towards on another when biased inwardly. And Fig. 26G shows a cross-sectional side view of cinching assembly **576** in which proximal collar **578** has uni-directional levers **580, 582** curved outwardly relative to one another when the levers are biased within collar **578.**

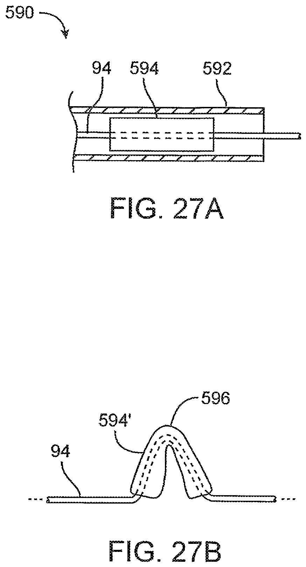

Figs. 27A and 27B shows yet another variation of cinching assembly **590** which utilizes a reconfigurable hollow member for cinching suture **94.** As shown in Fig. 27A, hollow member **594** may be constrained within tubular delivery member **592** to retain an elongate shape with suture **94** passing uninhibited therethrough. When the anchor is to be cinched, hollow member **594** may be advanced distally from tubular member **592** and when hollow member **594** has been ejected, it may adapted to reconfigure itself into a crimped configuration **594'** having a non-linear passageway. Suture **94** passing through the crimped configuration **594'** may be inhibited from passing freely therethrough by crimp **596** created within the hollow member. Hollow member **594** may have a variety of cross-sectional shapes, e.g., circular, rectangular, square, hexagonal, etc., and it is preferably made from a material having shape memory characteristics, e.g., Nitinol, such that when hollow member **594** is unconstrained, it may automatically reconfigure into its crimped configuration **594'.**

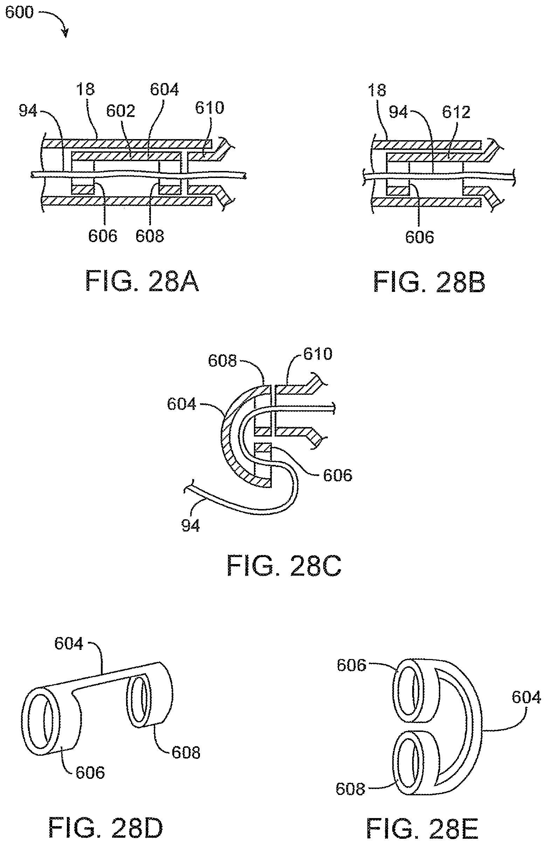

Another variation of cinching assembly **600** which is configured to reconfigure itself upon being unconstrained is shown in the cross-sectional views of Figs. 28A to 28C. In this variation shown in Fig. 28A, cinching collar **602** may comprise at least two circular members, first collar **606** and second collar **608,** connected by an elongate bridging member **604.** Cinching collar **602** may be positioned within launch tube **18** proximally adjacent to proximal collar **610** and adapted to reconfigure itself once released from launch tube **18** such that a tortuous path is created for suture **94.** Fig. 28B shows an alternative variation in the cinching collar which may be an integrated variation with the proximal collar such that first collar **606** is connected directly to the anchor via joining member **612.** In either variation, once the cinching collar has been ejected from launch tube **18,** the collar may configure itself such that first collar **606** and second collar **608** are biased towards one another to form, e.g., a "C"-shape as shown in Fig. 28C. The tortuous path which is created by cinching collar **602** for suture **94** to follow may be sufficient to prevent the further translation of the anchor relative to suture **94.** Figs. 28D and 28E, respectively, show perspective views of cinching collar **602** in a constrained delivery configuration and an unconstrained cinching configuration.

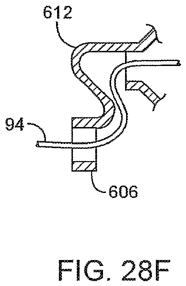

Fig. 28F shows a cross-sectional side view of another cinching assembly which is similar to the variation shown in Fig. 28B. Rather than having first collar **606** and joining member **612** reconfigure itself into a semi-circular shape relative to the anchor, first collar **606** may reconfigure itself to maintain its orientation relative to the anchor while joining member **612** may be formed to curve appropriately or approximately in an "S"-type configuration. The reconfigured cinching member may act to lock suture **94** relative to the anchor when the anchor is moved in a reverse direction.

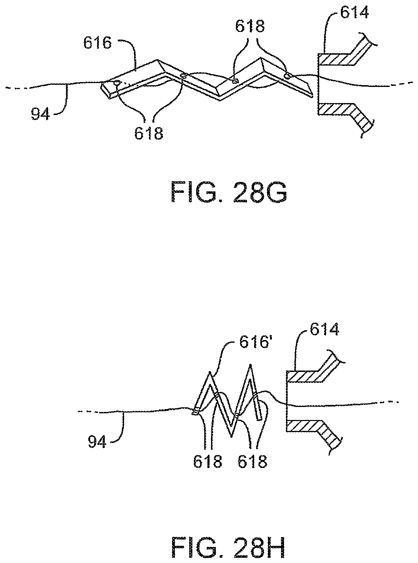

Another configuration for a cinching assembly is shown in the side view of Fig. 28G, which shows cinching member **616** located proximally of proximal collar **614.** Cinching member **616** may be fabricated from a variety of materials, e.g., Nitinol, spring stainless steel, etc., which exhibit shape memory or superelastic characteristics, or aspects thereof. In use, cinching member **616** may be configured into an elongate delivery configuration. When the tissue anchor is to be cinched or locked relative to the tissue, cinching member **616** may be released from a constraining force such that cinching member **616** reconfigures itself into an expanded or extended configuration which creates a tortuous path for suture **94** which sufficiently locks suture **94** within cinching member **616.**

Cinching member **616** may be comprised generally of an elongate bar, ribbon, cylinder, etc., or any elongate member having a diameter or cross-sectional area in its delivery configuration which is sufficiently small to be disposed and/or translated within launch tube **18.** Cinching member **616** may define a plurality of openings **618** along the length of cinching member **616** such that when cinching member **616** is in its elongate delivery configuration, as shown in Fig. 28G, suture **94** may be interwoven through openings **618** along a relatively straightened path. Openings **618** may be located along cinching member **616** at uniform locations or they may be randomly positioned along the length of cinching member **616.** When released, cinching member **616** may reconfigure itself into an expanded suture-locking configuration **616'** which is sufficiently large to prohibit its passage into or through proximal collar **614,** as shown in Fig. 28H. Expanded configuration **616'** may comprise any reconfigured shape so long as the expanded shape is adapted to create the tortuous path for suture **94** and is large enough so that passage through proximal collar **614** is not possible.

Other cinching and locking mechanisms which utilize mechanical clamping or crimping to achieve locking of the suture within or through the anchors may also be used to facilitate uni-directional locking.

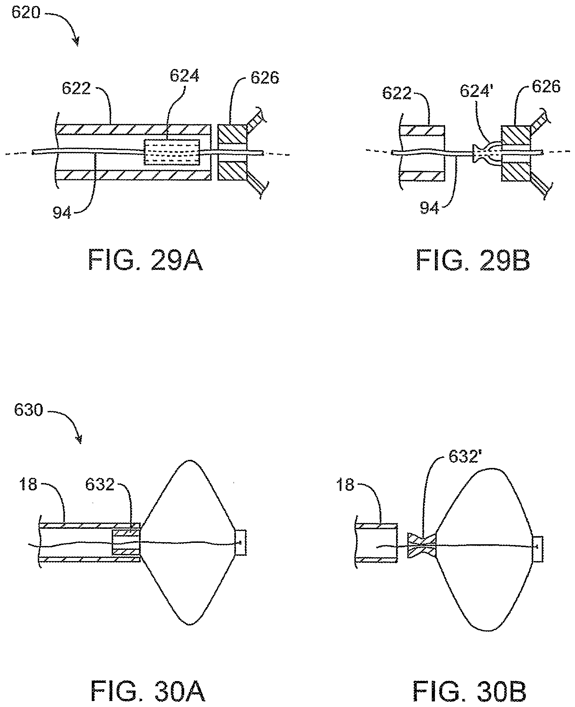

For instance, cinching assembly **620** may be seen in the cross-sectional view of Figs. 29A and 29B. Fig. 29A shows delivery tube member **622** having crimping collar **624** disposed therewithin proximally of anchor proximal collar **626.** Suture **94** may be passed through both crimping collar **624** and proximal collar **626.** Once the anchor has been desirably positioned, crimping collar **624** may be advanced distally adjacent to proximal collar **624** and mechanically crimped **624'** down upon suture **94** to create a lock and prevent the reverse movement of the anchor over suture **94,** as shown in Fig. 29B. The crimping may be accomplished via mechanical graspers or pinchers configured to clamp down upon collar **624.** Similarly, Fig. 30A shows cinching assembly **630** in which the crimping collar **632** may be integral with the anchor rather than being a separate member. Fig. 30B shows a mechanically crimped collar **632'** which eliminates the need for a separate collar.

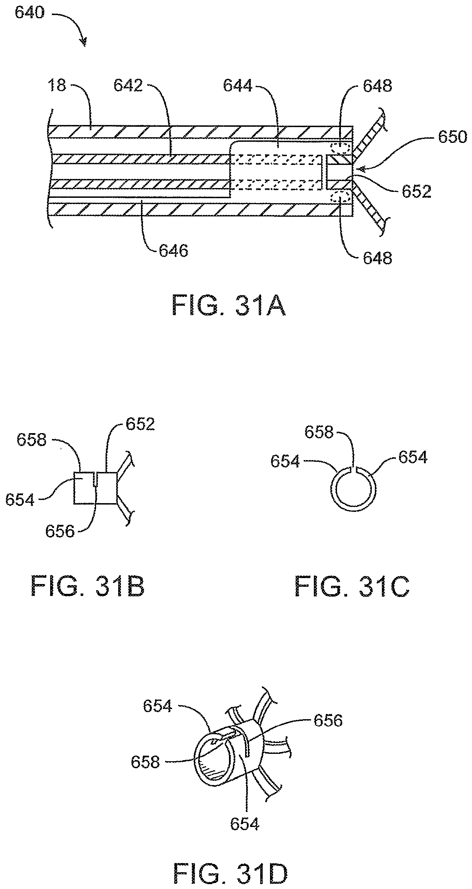

To accomplish mechanical crimping upon a cinching collar, various methods may be utilized. Fig. 31A shows one variation of a tool assembly **640** which may be adapted to apply a mechanical crimping force upon a crimping collar. As seen, launch tube **18** may have delivery push tube **642** located therewithin and positioned proximally of proximal collar **652** of the tissue anchor. Push tube **642** may be used to hold and/or eject proximal collar **652** from launch tube **18.** Crimping device **644** may be advanced within launch tube **18** via crimping control member **646,** which may be manipulated from its proximal end.

A collar retaining channel **650** may be defined in a distal end of crimping device **644** and adapted to receive and securely hold proximal collar **652** within during a clamping or crimping process. Crimping members or arms **648** may be positioned within crimping device **644** on either side of retaining channel **650.** When proximal collar **652** or crimping sleeve is to be clamped or crimped, crimping members or arms **648** may be driven into contact with proximal collar **652** to crimp the collar. Moreover, crimping arms **648** may be actuated through a variety of methods, e.g., hydraulically, pneumatically, via mechanical leverage, etc.

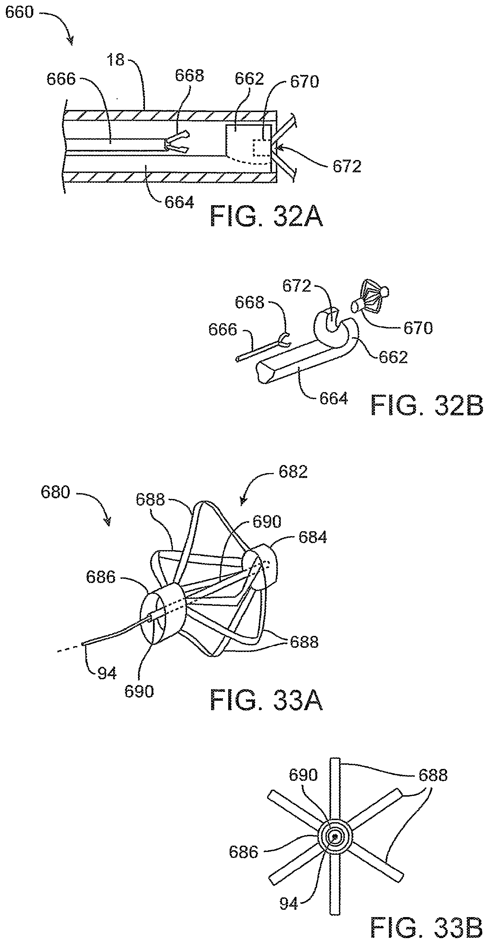

An alternative crimping assembly **660** is shown in the cross-sectional view of Fig. 32A. Crimping device **662** may be seen within launch tube **18** extending from crimping control member **664.** Collar retaining channel **672** may be likewise defined within crimping device **662** for retaining proximal collar **670** during a crimping procedure. This variation may utilize a separate elongate crimping member **666** having actuatable crimping arms **668** positioned at a distal end of elongate member **666.** In use, with proximal anchor collar **670** positioned within retaining channel **672,** elongate member **666** may be advanced distally until crimping arms **668** are positioned over proximal collar **670** and crimped down. Fig. 32B shows an exploded perspective view of the crimping assembly.

Figs. 31B to 31D show side, end, and perspective views, respectively, of one variation of an anchor proximal collar **652** which is adapted for crimping upon a suture passing therethrough. To facilitate crimping of the collar **652,** a circumferential slot **656** may be defined through collar **652** partially around its circumference. Another longitudinal slot **658** may be defined through collar **652** extending longitudinally from a proximal edge of collar **652** to circumferential slot **656.** These slots **656, 658** may define at least two crimping arms **654** which may be crimped down upon a length of suture passing through collar **652.**

Aside from the crimping mechanisms described above, additional measures may be optionally implemented to facilitate the cinching or locking of an anchor. Other measures may also be taken to inhibit any damage from occurring to the suture routed through an anchor.

To ensure that the integrity of suture **94** is maintained in the presence of metallic basket anchors **682** and to ensure that suture **94** is not subjected to any nicks or cuts, the portion of suture **94** passing through basket anchor **682** may be encased in a protective sleeve **690,** as shown in the perspective view of Fig. 33A of anchor-sleeve assembly **680.** The basket anchor **682** is shown in this variation as having anchor struts or arms **688** in a partially deployed configuration. Sleeve **690** may extend between distal collar **684** and proximal collar **686** to prevent excessive contact between suture **94** and elements of basket anchor **682.** Fig. 33B shows an end view of the anchor-sleeve assembly **680** showing the relative positioning of sleeve **690** relative to suture **94** and anchor collar **686.** Sleeve **690** may be made from a variety of polymeric materials, e.g., polypropylene, PTFE, etc., provided that the material is suitably soft.

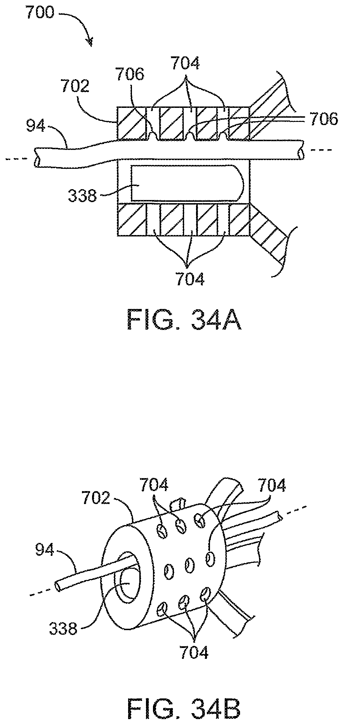

Fig. 34A shows a cross-sectional view of cinching assembly **700** which may be implemented with any of the cinching and locking mechanisms described above. This particular variation utilizes the partial cold-flowing of the engaged suture **94** to enhance the locking or cinching effect of the tissue anchor. The cinching collar, or in this variation proximal collar **702,** against which suture **94** is wedged may have multiple through-holes **704** defined over the surface of collar **702.** The cross-sectional side view shows suture **94** wedged within collar **702** against locking pin **338.** The portion of suture **94** which is adjacent to through-holes **704** may have regions which cold-flow partially into through-holes **704,** as shown by cold-flowed suture material **706.** These portions of suture material **706** may enhance the locking aspects of suture **94** against collar **702.** Fig. 34B shows a perspective view of collar **702** with multiple through-holes **704** defined over the body of collar **702.** Through-holes **704** may be defined in a uniform pattern; alternatively, they may be randomly defined over collar **702** or only over portions of collar **702.**

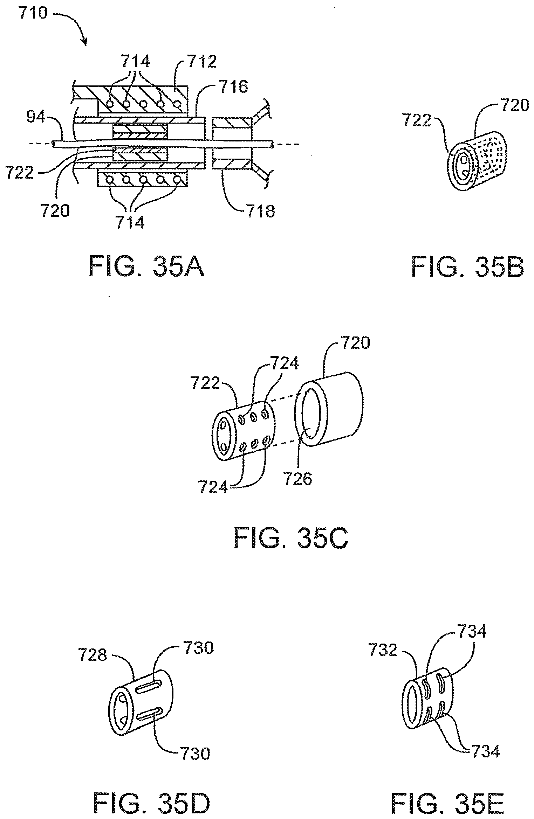

Figs. 35A to 35E show an alternative variation **710** for locking a tissue anchor relative to suture **94.** An outer sleeve **720** which is preferably comprised of a polymeric material capable of at least partially flowing when heated, e.g., PTFE, may be disposed circumferentially about an electrically conductive inner sleeve **722.** As shown in the perspective views of Figs. 35B and 35C, inner sleeve **722** maybe disposed within lumen **726** of outer sleeve **720.** Inner sleeve **722** may randomly or uniformly define a plurality of openings or through-holes **724** over the surface of inner sleeve **722.**

In operation, outer and inner sleeves **720, 722,** respectively, may be positioned within delivery push tube **716** proximally of proximal collar **718** with suture **94** passing therethrough. When the tissue anchor has been desirably positioned and suture **94** has also been desirably tensioned, an induction unit **712** having one or more induction coils **714** therewithin may be positioned circumferentially (or at least partially circumferentially) about outer and inner sleeves **720, 722.** Induction unit **712** may be configured to be disposed within the launch tube **18** or it may be configured to be advanced over or positioned upon launch tube **18.** Thermal energy or electrical energy in various forms, e.g., RF, microwave, etc., may be delivered to induction coils **714** such that the energy heats inner sleeve **722,** which may be positioned within induction coils **714,** as shown in Fig. 35A. As inner sleeve **722** is heated via induction, the inner surface of outer sleeve **720** may be partially melted or deformed such that the material flows at least partially through or within through-hole **724** and contacts suture **94** positioned within inner sleeve **722.** The flowed material may cool and act to lock outer and inner sleeves **720, 722** onto suture **94.** Induction unit **712** may then be removed from the area leaving outer and inner sleeves **720, 722** locked relative to the tissue anchor.

Although inner sleeve **722** shows through-holes **724** as circularly defined openings, other shapes may be utilized. For example, Fig. 35D shows a perspective view of one inner sleeve variation **728** having longitudinally defined slots **730.** Alternatively, Fig. 35E shows a perspective view of another inner sleeve variation **732** having circumferentially defined slots **734.** Any variety of opening shapes may be utilized so long as the opening or openings allow for material from the outer sleeve **720** to flow through into contact with the suture positioned within.

Other configurations for the anchor itself may also be utilized in conjunction with any of the cinching apparatus described herein. For instance, the linear cinching member **616** shown in Figs. 28G and 28H may also be utilized to function as an anchor itself. Such a linear anchor may be fabricated from a metal, e.g., Nitinol, spring stainless steel, etc., which exhibit shape memory or superelastic characteristics, as described above.

Alternatively, an elongate linear anchor may be comprised of a flexible polymeric material such that the anchor remains in a linear configuration when disposed within a delivery tube for deployment. Examples of potential polymeric materials may include, e.g., polyethylene, polyester, polystyrene, polycarbonate, nylon, teflon, elastomers, etc. However, once the elongate anchor is ejected or deployed from the delivery tube, the anchor may be configured to compress longitudinally, i.e., along the length of the anchor, upon the application of a force in a longitudinal direction relative to the anchor such that portions of the linear anchor bow or expand in a radial direction. The anchors may be configured to bow or expand through a number of techniques such as heat-setting the material. Additional methods and variations are described below in further detail.

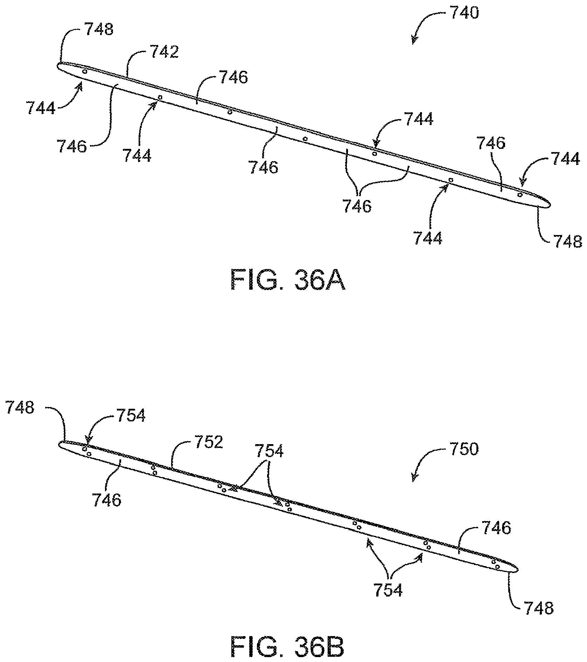

Fig. 36A shows a perspective view of one variation **740** of a linear anchor. In this variation, the linear anchor **740** may generally comprise an elongate ribbon or flattened wire **742** having at least one or more openings **744** defined through the anchor **740** along its length. Linear anchor **740** may generally range anywhere in length from, e.g., 8.99 to 18.415 cm (3.50 to 7.25 inches), or greater. The thickness of linear anchor **740** may also range from, e.g., 0.0254 to 0.0508 cm (0.010 to 0.020 inches), and is preferably 0.03302 cm (0.013 inches), while the width may range from, e.g., 0.0762 to 0.1651 cm (0.030 to 0.065 inches), and is preferably 0.127 cm (0.050 inches).

Ribbon **742** illustrates openings **744** in an optionally offset pattern relative to one another along the length to enhance the ribbon **740** twisting or bowing effect when compressed linearly. Alternatively, openings **744** may be linearly aligned along the length, if desired. Moreover, this variation illustrates openings **744** uniformly spaced apart from one another anywhere from, e.g., 0.9525 to 1.905 cm (0.375 to 0.750 inches), over the length of anchor 740. Alternatively, openings **744** may vary in diameter, e.g., 0. 0381 cm (0.015 inches), so long as the length of suture being routed or interwoven through openings **744** is able to freely slide through openings **744.**

One or both ends **748** of ribbon **742** may be optionally configured to be atraumatic by having a blunted end or a tapered end to reduce or inhibit damage to the surrounding tissue. The regions **746** of ribbon **742** located between openings **744** may be configured to flex in a predetermined orientation when anchor **740** is linearly compressed such that the compressed and expanded ribbon **742** expands in a predetermined orientation, as described further below.

Fig. 36B shows a perspective view of another variation **750** of ribbon **752** which is similar to variation **740** of Fig. 36A. Variation **750** defines a plurality of openings **754** which may be defined in a varied offset pattern, e.g., pairs of openings **754** may be offset relative to one another, as shown.

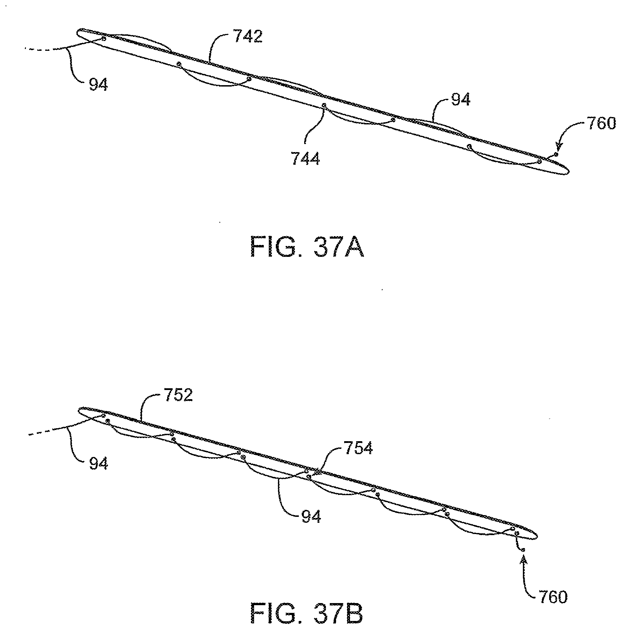

In order to ensure the collapse of the ribbon into a bowed or expanded pattern, the suture may be routed through each, or at least several, of the openings. Fig. 37A shows the ribbon **742** of Fig. 36A with suture **94** routed through each opening **744** in an alternating or interwoven pattern. Similarly, Fig. 37B shows the ribbon **752** of Fig. 36B with suture **94** routed through openings **754** likewise in an alternating or interwoven pattern. In either case, a terminal end of suture **94** may be formed into a knot **760** distally of the ribbon to prevent the passage of suture **94** proximally. This may be done to facilitate the collapse of the ribbon by simply pulling or tensioning suture **94** to collapse the ribbon upon itself. Rather than forming a knot **760,** suture **94** may be attached to the ribbon through any variety of methods, e.g., suture **94** may be tied or welded to the ribbon, or it may simply be attached via an adhesive, etc. Accordingly, when the ribbon is compressed in a longitudinal direction relative to suture **94** by tensioning suture **94,** the ribbon compresses into an expanded pattern while bending or folding along the regions in-between the openings.

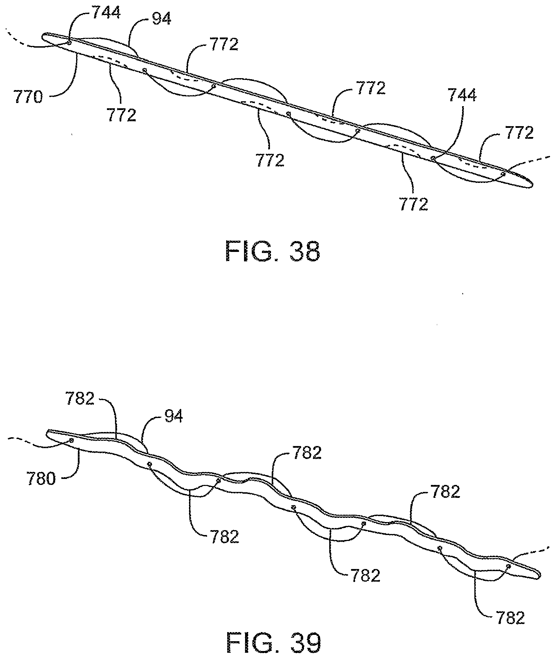

Various methods may be utilized to facilitate the bending or folding of the ribbon anchor into desired expanded patterns. One variation may include an elongate ribbon **770** having alternating portions **772** of the ribbon material between the openings **744** notched out or removed, as shown in the variation of Fig. 38. Alternatively, Fig. 39 shows another variation in which the ribbon **780** may be formed to define undulations **782** such that an "S"-type ribbon pattern is fabricated.

Once the ribbon anchor is urged into its collapsed and deployed configuration, the ribbon anchor may be further configured to provide some degree of spring force which permits the anchor to absorb a range of deflections which may be imparted upon the anchor. The ability of the ribbon anchor to absorb a range of deflections may allow the ribbon anchor to absorb some force which would otherwise be imparted to the underlying tissue surface.

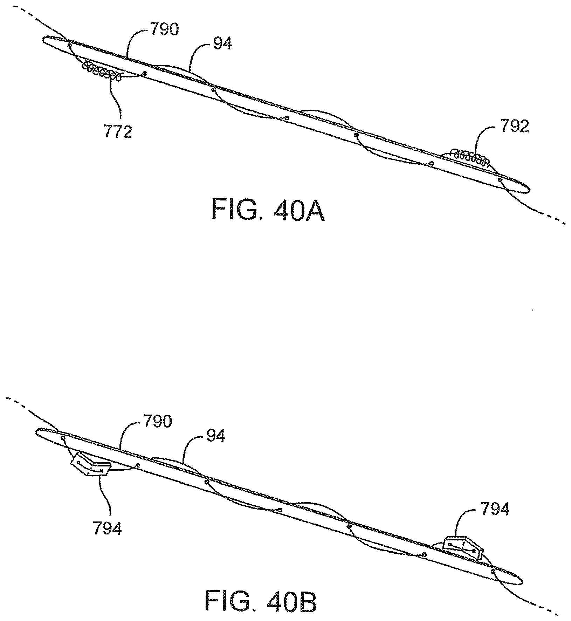

Such a spring force may be imparted to the ribbon anchor in one variation as shown in Fig. 40A, which shows a perspective view of ribbon anchor **790** with suture **94** routed through the openings. Biasing elements, such as springs **792,** may be positioned along portions of ribbon anchor **790** with suture **94** routed therethrough. The variation shows two springs **792** positioned near either end of ribbon **790;** however, a single spring or multiple springs may be utilized depending upon the desired elastic spring effects of the ribbon anchor **790.** Moreover, the spring or springs **792** may be positioned at any position along the length of the ribbon anchor **790;** alternatively, one or more springs **792** may even be positioned proximally or distally of one or both ends of the ribbon anchor **790** rather than between the folds of the ribbon anchor **790.**

Fig. 40B shows a variation in ribbon anchor **790** which is similar to the assembly of Fig. 40A. This variation utilizes biased elements **794,** which may be fabricated from one of a variety of metallic or polymeric materials similar to or the same as the material of ribbon anchor **790.** Biased elements **794** may generally comprise angled members having suture **94** routed therethrough and which are biased to pivot about a hinge and retain its angled configuration.

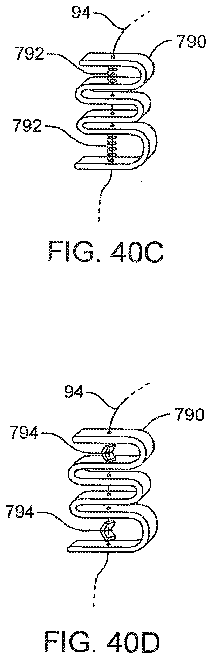

In use, when suture **94** is tensioned or pulled relative to ribbon anchor **790,** ribbon anchor **790** may reconfigure itself into its compressed configuration. Fig. 40C shows a perspective view of the ribbon **790** of Fig. 40A which has been partially collapsed. As shown, springs **792** may be compressed between the portions of ribbon anchor **790** and function to provide a biasing force which allows the anchor assembly to absorb a range of deflections while isolating the force from the underlying tissue. Fig. 40D shows the partially compressed anchor ribbon **790** with the biased elements **794** from Fig. 40B. This variation of the anchor assembly may function similarly to that of the assembly of Fig. 40C.

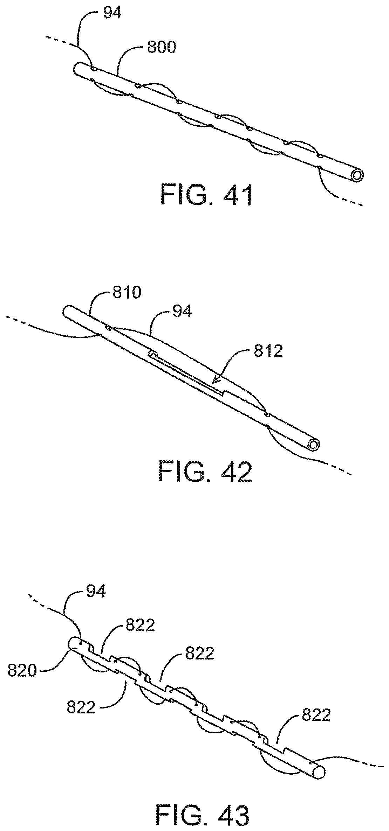

An alternative variation of the ribbon anchor is shown in the perspective view of Fig. 41. Rather than utilizing a ribbon or flattened wire, this variation utilizes a tubular member **800** with suture **94** routed through a plurality of openings defined along its length. The tubular member **800** may be fabricated from any of the materials as described above, and because of its circular cross-section, it may provide some spring force as tubular member **800** is collapsed upon itself. Other alternatives may also utilize elongate members having elliptical, rectangular, triangular, etc., cross-sections.

Fig. 42 shows a perspective view of tubular member **810** having a partial cut-out **812** along the length of tubular member **810.** A single cut-out may be utilized to provide for a bending portion. Alternatively, multiple cut-outs **822** may be formed in a tubular member **820,** as shown in Fig. 43. Multiple cut-outs **822** may be formed along a single side of tubular member **820,** or they may be alternated on opposing sides, as shown in the figure. In either case, suture **94** may be routed in an alternating pattern along the lengths of the tubular members to facilitate the bending or folding of the members, as described above.

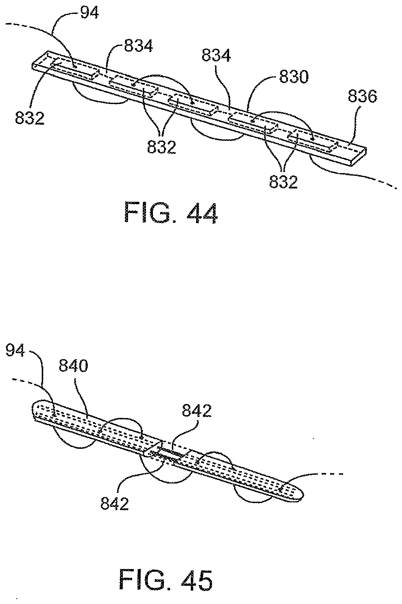

Another alternative ribbon anchor **830** is shown in Fig. 44, which illustrates multiple individual lengths of elements **832** encased (or at least partially encased) in a coating or covering **836.** Each or a number of the elements **832** may be fabricated from a variety of materials, e.g., metals such as Titanium, stainless steel, Nitinol, etc., or plastics, etc., and the coating or covering **836** may be made from a variety of flexible polymeric or elastomeric materials. The length of the coating or covering **836** extending between each of the elements **832** may function as living hinges **834** while suture **94** may be routed through each or several of the elements **832.** When ribbon anchor **830** is compressed, it may fold along the living hinges **834** as described above.

Fig. 45 shows yet another alternative in which ribbon anchor **840** may be comprised of one or more lengths of wire **842** covered or coated with the same or similar material as coating or covering **836** above. Wires **842** may be fabricated from the same or similar material as elements **832** above. Similarly, suture **94** may be routed through a plurality of openings defined along the length of ribbon anchor **840.**

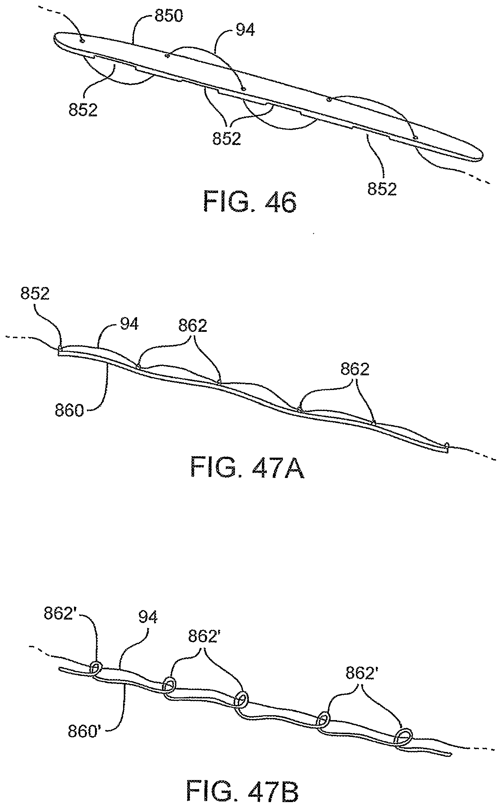

Fig. 46 shows yet another variation of ribbon anchor **850** which is similar to the ribbon anchors described above, yet this variation may have a length which has a non-uniform thickness. For instance, ribbon anchor **850** may have one or more regions **852** which are notched out or thinned relative to the rest of the length of ribbon anchor **850.** The thicknesses of the notched or thinned regions **852** may be varied to alter the bending or folding characteristics of ribbon anchor **850,** as desired.

Fig. 47A shows yet another variation in which a length of wire **860** may simply be utilized to fold or flatten in an expanded pattern. Wire **860** may be extruded from various materials, e.g., stainless steel, Nitinol, Titanium, plastic, etc., and it may be further configured to have shape memory characteristics such that when collapsed, wire **860** folds or collapses into a predetermined pattern. Wire **860** may also comprise one or more eyelets **862** along its length through which suture **94** may be routed. Eyelets **862** may be welded or adhered to wire **860** through any number of fastening methods. Another alternative is shown in Fig. 47B, which is similar to the variation of Fig. 47A. In this variation, a single wire **860',** similar to above, may be configured to define a plurality of loops **862'** rather than having separate eyelets attached thereto. Suture **94** may be routed through these formed loops **862'.**

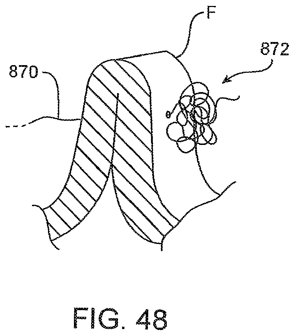

Alternatively, a length of wire **870** may be utilized without suture. Fig. 48 shows an example in which wire or ribbon **870** may be comprised of a shape memory alloy, such as Nitinol, which is configured to form a tangled portion **872** of wire **870** when unconstrained, similar to a "bird's nest". This tangled portion **872** may simply expand into an intertwined mass of wire which prevents its passage through a tissue fold F.

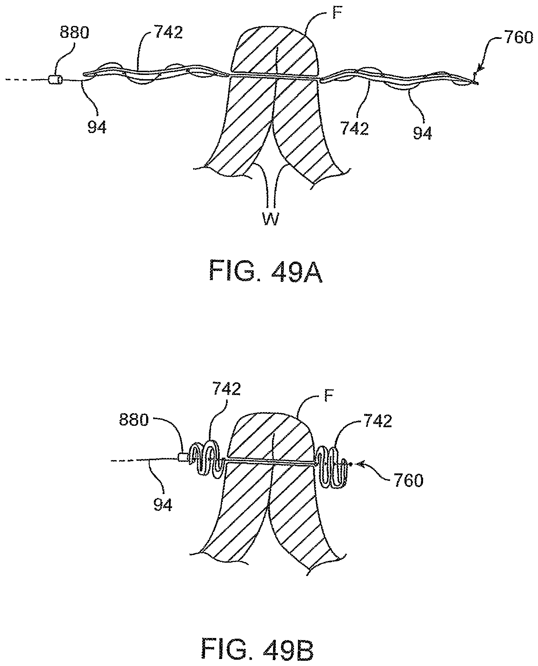

In operation for any of the ribbon anchors described above, one or more ribbon anchors may be utilized. For instance, a first anchor may be positioned distally of a tissue fold **F** and a second anchor may be positioned proximally of the same tissue fold **F,** as shown in Fig. 49A. The distal portion of suture **94,** which extends between the two anchors, may be anchored to the first anchor via, e.g., fastener or knot **760.** As suture **94** is tensioned or pulled, each of the ribbon anchors **742** may be compressed and collapse into an expanded configuration against the tissue fold **F.** A locking mechanism **880,** which may comprise any of the locking mechanisms described above, may be proximally positioned over suture **94** and cinched against the ribbon anchor **742** to thereby lock each of the ribbon anchors **742** into their collapsed and expanded configurations, as shown in Fig. 49B. Although this example illustrates the use of ribbon anchors **742,** any of the linear anchors described above may be utilized either alone or in combination with any other linear anchor or other anchor described above.

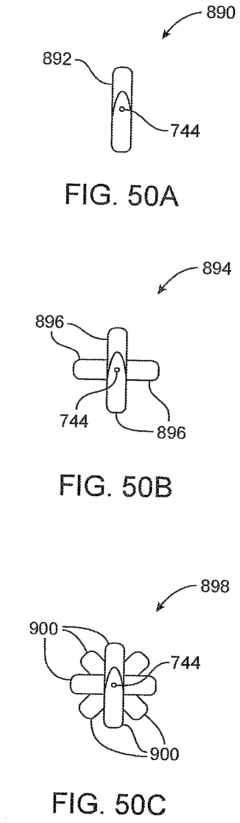

As mentioned above, the ribbon anchor may be collapsed into an expanded configuration against the tissue surface. Accordingly, the ribbon anchor may be configured to collapse in a variety of bowed or expanded configurations such that the contact area of the anchor against the tissue surface is increased, if so desired. Fig. 50A shows a top view of one variation of a ribbon anchor **890** which is configured simply to fold linearly upon itself to form arms **892.**

Fig. 50B shows a top view of another variation **894** in which the ribbon anchor may be configured to form a crossing pattern with at least four arms **896.** And Fig. 50C shows a top view of yet another ribbon anchor **898** where multiple arms **900** may be formed in a radial pattern to facilitate the distribution of the anchor against the tissue surface.

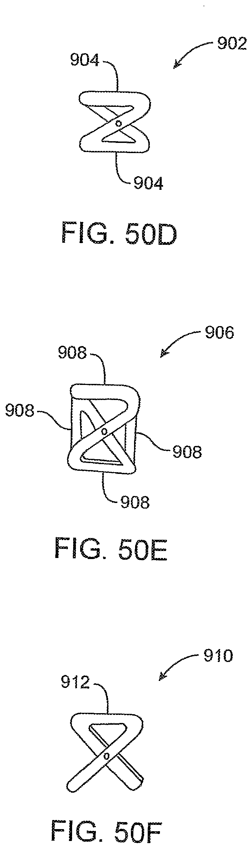

Alternatively, the ribbon anchor may be configured to form a number of secondary support arms **904,** as shown in the bowtie-like configuration of variation **902** in Fig. 50D. Fig. 50E shows another variation **906** where a square configuration may be formed via multiple support arms **908** formed by the collapsing anchor. Fig. 50F shows yet another variation **910** where a single support arm **912** may be formed by the collapsing anchor structure. Any number of patterns and folded arms may be formed depending upon the desired configuration and aspects of the collapsed ribbon anchor.

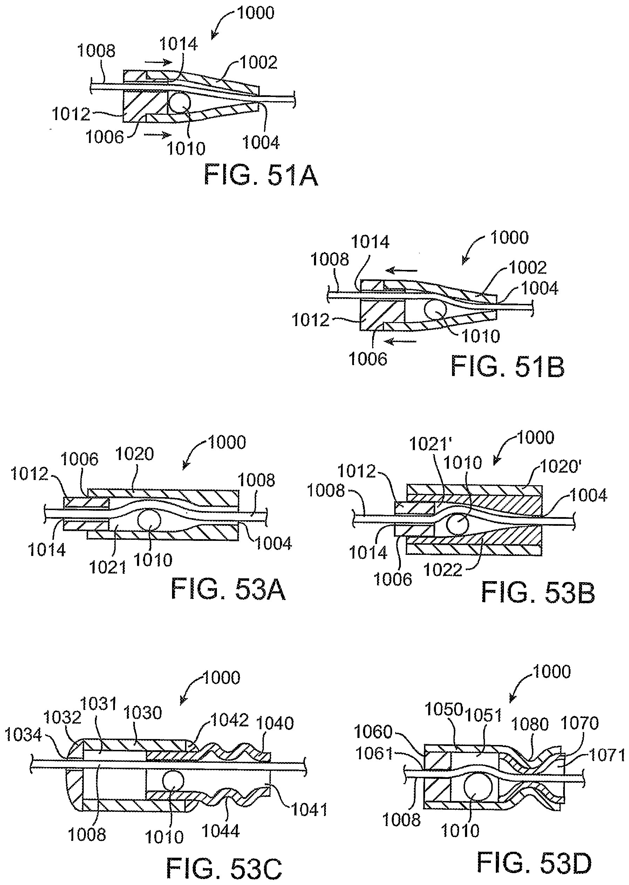

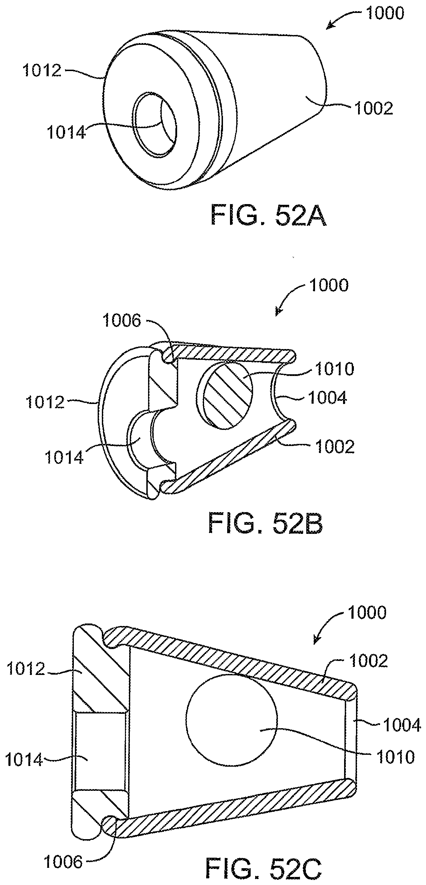

Referring now to Figs. 51 and 52, a cinching assembly comprising an interference element is described. Cinching assembly **1000** comprises tubular or hollow body member **1002** having distal opening **1004** and proximal opening **1006.** If body member **1002**is tubularly or cylindrically shaped, the passage defined through body member **1002** is preferably of a tapered or conical shape. Body member **1002** may optionally be shaped into a tapered or conical configuration. Distal opening **1004** is sized for passage of suture **1008** therethrough, while proximal opening **1006** is sized for passage of both the suture and interference element **1010,** illustratively a ball or sphere.

Assembly **1000** further comprises proximal plug or cap **1012** that is coupled to the proximal end of body member **1002** and that fills proximal opening **1006** of the body member. Plug or cap **1012** may be fitted or held within body member **1002** through a variety of methods. For instance, plug or cap **1012** may be friction-fitted within the proximal opening **1006.** Alternatively, plug or cap **1012** may be fitted via a circumferential detent configured to snap into place within the proximal opening **1006;** and in yet another example, plug or cap **1012** may simply be held within or adjacent to proximal opening **1006** via an adhesive. As best seen in Figs. 52, plug **1012** comprises through-hole **1014** configured for passage of suture **1008** (for the purposes of illustration, suture **1008** is not shown in Figs. 52).

Ball **1010** may, for example, have a diameter between about 0.020" and 0.040", although any other diameter may be provided as desired. Furthermore, the ball may be fabricated from any of a variety of materials, such as aluminum, titanium or steel; alternative materials will be apparent. Suture **1008** may, for example, have a nominal diameter of about 0.016", although other diameters may be provided. Distal opening **1004** of body member **1002** and through-hole **1014** of proximal plug **1012** may, for example, have a diameter of about 0.018", although other diameters may be provided.

As illustrated by arrows in Figs. 51A, urging body member **1002** distally, i.e., in the direction of the arrows shown, relative to suture **1008** urges ball **1010** towards the proximal end of body member **1002** and against plug or cap **1012.** In this example, since the proximal end of the conical body member may have a larger cross-section than its tapered distal end, suture **1008** may pass freely through cinching assembly **1000,** thereby allowing the suture to be cinched. As illustrated by arrows in Fig. 51B, moving body member **1002** proximally relative to suture **1008** urges ball **1010** towards the tapered distal end of body member **1002.** This interference or friction locks suture **1008** between ball **1010** and the interior wall of body member **1002,** thereby allowing uni-directional cinching of assembly **1000** relative to suture **1008** by locking cinching assembly **1000** and preventing any reverse movement of suture cinching.

Referring now to Figs. 53, variations of cinching assembly **1000** are described. In the variation of Fig. 53A, conical body **1002** has been replaced with tubular body **1020** having tapered or profiled interior lumen **1021,** which facilitates friction locking of suture **1008** between ball **1010** and the profiled wall of the lumen. Body **1020** may, for example, be machined, cast or molded to achieve the profile of lumen **1021.** Proximal plug **1012** illustratively comprises a hypotube concentrically disposed within body **1020.** Through-hole or lumen **1014** of plug or hypotube **1012** is sized for passage of suture **1008** therethrough, but is smaller than the diameter of ball **1010** to prevent ball **1010** from exiting or falling out of body **1020.**

Fig. 53B provides a similar variation of assembly **1000** having body **1020'** with profiled interior lumen **1021'.** In contrast to the variation of Fig. 53A, body **1020'** may, for example, comprise a standard hypotube, while the profile of lumen **1021'** may be formed via secondary insert **1022** disposed within the hypotube. Insert **1022** may, for example, comprise epoxy injected within the hypotube, or may comprise a separate piece that is friction fit or otherwise fit within the hypotube.

In the variation of Fig. 53C, assembly **1000** comprises tubular body **1030** having lumen **1031,** as well as end cap or plug **1032** with proximal opening **1034** for passage of suture **1008.** The end cap may, for example, comprise a bead of epoxy, while the tubular body may comprise a hypotube. Crimped hypotube **1040** is concentrically disposed over or within tubular body **1030** and is coupled to body **1030** at attachment **1042.** Attachment **1042** may, for example, comprise a weld formed between the tubular body and the hypotube, or may comprise a glue attachment, such as a cyanoacrylate attachment, etc. Hypotube **1040** further comprises central crimp **1044.** The reduced diameter of lumen **1041** through hypotube **1040** in the vicinity of crimp **1044** facilitates friction locking of ball **1010** with suture **1008** to prevent reversal of suture cinching.

In the variation of Fig. 53D, cinching assembly **1000** is formed from three tubes, e.g., hypotubes. Proximal hypotube **1060** is concentrically disposed within a proximal end of body hypotube **1050,** while distal hypotube **1070** is disposed within a distal end of the body hypotube. Body hypotube **1050** comprises lumen **1051** configured for passage of ball **1010** and suture **1008** therethrough; while proximal hypotube **1060** comprises lumen **1061,** and distal hypotube **1070** comprises lumen **1071,** both of which are configured for passage of suture **1008** therethrough, but not for passage of ball **1010.** Body hypotube **1050** and distal hypotube **1070** comprise crimp **1080** that locally reduces lumens **1051** and **1071.** As seen in Fig. 53D, the ball and suture are disposed within lumen **1051** in a manner that allows free proximal movement of suture **1008** relative to cinching assembly **1000,** but that friction locks suture **1008** between ball **1010** and crimp **880** during distal movement of the suture relative to the assembly.

The cinching assembly variations of Figs. 51-53 rely on an interference fit and/or friction between a suture, thread or other flexible element; an interference element, such as a ball or sphere; and an interior surface of a body member element through which the flexible element and the interference element pass. The material characteristics and/or surface texture or roughness of these three elements may be specified to achieve, for example, desired frictional characteristics and interactions, ease of cinch, locking forces, etc. Exemplary materials and surface characteristics for any or all of the cinching assembly elements include, but are not limited to, metals, polymers, elastomers, sandblasted metals, smooth metals, sapphire, glass, etc. Additional materials and surface characteristics will be apparent to those of skill in the art.

Although a number of illustrative variations are described above, it will be apparent to those skilled in the art that various changes and modifications may be made thereto without departing from the scope of the invention as defined by the appended claims. Moreover, although specific locking or cinching configurations may be shown with various types of anchors, it is intended that the various locking or cinching configurations be utilized with the various types of anchors in various combinations as practicable.

## Claims

1. An anchor for implantation in a body, comprising:
an anchor body (64, 112, 132) adapted to be delivered through a hollow member or delivery device (18) for placement against a tissue surface (F); and
a locking mechanism (88, 118, 546) adapted to be delivered through the hollow member or delivery device (18), wherein the locking mechanism comprises a body having a proximal end and a distal end, and defining a lumen for passage of a flexible member (70, 94) therethrough;
wherein the locking mechanism comprises at least two arms (550, 552), wherein a first arm of the at least two arms is arranged nearer to the proximal end of the body and a second arm of the at least two arms is arranged nearer to the distal end of the body, and wherein the at least two arms are biased to project in opposing directions into the lumen to cinch the flexible member (70, 94) by creating a tortuous path such that the flexible member (70, 94) is uni-directionally advanceable through the lumen relative to the anchor body (64, 112, 132).

2. The anchor of claim 1 wherein the anchor body comprises a basket anchor (306).

3. The anchor of claim 2 wherein the basket anchor (306) is reconfigurable from a delivery configuration within the hollow member or delivery device to an expanded configuration outside the hollow member.

4. The anchor of any preceding claim wherein the hollow member or delivery device is selected from the group consisting of endoscopes, hollow members, tubular members, catheters, needles, and trocars.

5. The anchor of any preceding claim wherein the flexible member (70, 94) comprises a length of suture.

6. The anchor of claim 5 wherein the suture comprises a plurality of knots or protrusions (170) along the length of the suture.

7. The anchor of claim 6 wherein the knots or protrusions (170) are formed at regular intervals along the length.

8. The anchor of claim 6 wherein the knots or protrusions (170) are formed intermittently along the length.

9. The anchor of any one of claims 5 to 8, wherein the length of suture comprises a monofilament, multifilament, elastic, or elastomeric material.

10. The anchor of any one of claims 5 to 8, wherein the length of suture comprises a metallic material, preferably wherein the metallic material is selected from the group consisting of Nitinol, stainless steel, and Titanium.

11. The anchor of any one of claims 5 to 10, wherein at least a portion of the length of suture is surrounded by a material having a frictional coefficient higher than the suture.

12. The anchor of claim 11 wherein the material comprises a metal coating or sleeve (181).

13. The anchor of any preceding claim wherein the anchor body (64, 112, 132) defines a tapered opening for passage of the flexible member therethrough.

14. The anchor of any preceding claim wherein the locking mechanism (64, 88, 118) is contained within the anchor body (64, 112, 132).

15. The anchor of any preceding claim wherein the locking mechanism (64, 88, 118) is in-line with the flexible member (70, 94).

16. The anchor of any preceding claim wherein the locking mechanism (64, 88, 118) is adapted to automatically impart a locking force upon the flexible member (70, 94).

17. The anchor of any preceding claim wherein the arms (118, 138, 140) are biased to cinch the flexible member (70, 94) against a surface of the lumen.

18. The anchor of claim 17 wherein the arms (118, 138, 140) are further adapted to release the flexible member (70, 94) when the anchor body is advanced distally.

19. The anchor of any preceding claim wherein the arms (118, 138, 140) are biased to project at an angle proximally of the anchor body.

20. The anchor of claim 19 wherein the arms (118, 138, 140) define a tapered opening through which the flexible member (70, 94) is passed.

21. The anchor of claim 19 wherein the arms (118, 138, 140) are integral with the anchor body (64, 112, 132).

22. The anchor of any preceding claim, wherein the locking mechanism is positioned proximally of the anchor body.

## Patentansprüche

1. Anker zur Implantation in einen Körper, der Folgendes umfasst:
einen Ankerkörper (64, 112, 132), der dafür ausgelegt ist, durch ein hohles Element oder eine Abgabevorrichtung (18) zur Platzierung gegen eine Gewebeoberfläche (F) abgegeben zu werden; und
einen Verriegelungsmechanismus (88, 118, 546), der dafür ausgelegt ist, durch das hohle Element oder die Abgabevorrichtung (18) abgegeben zu werden, wobei der Verriegelungsmechanismus einen Körper mit einem proximalen Ende und einem distalen Ende umfasst und ein Lumen für den Durchgang eines flexiblen Elements (70, 94) dort hindurch definiert;
wobei der Verriegelungsmechanismus mindestens zwei Arme (550, 552) umfasst, wobei ein erster Arm der mindestens zwei Arme näher am proximalen Ende des Körpers angeordnet ist und ein zweiter Arm der mindestens zwei Arme näher am distalen Ende des Körpers angeordnet ist, und wobei die mindestens zwei Arme vorgespannt sind, um in entgegengesetzte Richtungen in das Lumen zu ragen, um das flexible Element (70, 94) durch Erzeugen eines gewundenen Pfades festzuhalten, so dass das flexible Element (70, 94) durch das Lumen relativ zum Ankerkörper (64, 112, 132) unidirektional vorschiebbar ist.

2. Anker nach Anspruch 1, wobei der Ankerkörper einen Korbanker (306) umfasst.

3. Anker nach Anspruch 2, wobei der Korbanker (306) von einer Abgabekonfiguration innerhalb des hohlen Elements oder der Abgabevorrichtung zu einer erweiterten Konfiguration außerhalb des hohlen Elements umkonfigurierbar ist.

4. Anker nach einem der vorhergehenden Ansprüche, wobei das hohle Element oder die Abgabevorrichtung aus der Gruppe ausgewählt ist, die aus Endoskopen, hohlen Elementen, Rohrelementen, Kathetern, Nadeln, und Trokaren besteht.

5. Anker nach einem der vorhergehenden Ansprüche, wobei das flexible Element (70, 94) eine Nahtlänge umfasst.

6. Anker nach Anspruch 5, wobei die Naht mehrere Knoten oder Vorsprünge (170) entlang der Nahtlänge umfasst.

7. Anker nach Anspruch 6, wobei die Knoten oder Vorsprünge (170) in regelmäßigen Abständen entlang der Länge gebildet sind.

8. Anker nach Anspruch 6, wobei die Knoten oder Vorsprünge (170) intermittierend entlang der Länge gebildet sind.

9. Anker nach einem der Ansprüche 5 bis 8, wobei die Nahtlänge ein Monofilament-, Multifilament-, elastisches, oder elastomeres Material umfasst.

10. Anker nach einem der Ansprüche 5 bis 8, wobei die Länge der Naht ein metallisches Material umfasst, wobei das metallische Material vorzugsweise aus der Gruppe bestehend aus Nitinol, rostfreiem Stahl, und Titan ausgewählt ist.

11. Anker nach einem der Ansprüche 5 bis 10, wobei mindestens ein Teil der Länge des Nahtmaterials von einem Material umgeben ist, dessen Reibungskoeffizient höher als das Nahtmaterial ist.

12. Anker nach Anspruch 11, wobei das Material eine Metallbeschichtung oder -hülse (181) umfasst.

13. Anker nach einem der vorhergehenden Ansprüche, wobei der Ankerkörper (64, 112, 132) eine sich verjüngende Öffnung zum Durchgang des flexiblen Elements dort hindurch definiert.

14. Anker nach einem der vorhergehenden Ansprüche, wobei der Verriegelungsmechanismus (64, 88, 118) in dem Ankerkörper (64, 112, 132) enthalten ist.

15. Anker nach einem der vorhergehenden Ansprüche, wobei der Verriegelungsmechanismus (64, 88, 118) mit dem flexiblen Element (70, 94) ausgerichtet ist.

16. Anker nach einem der vorhergehenden Ansprüche, wobei der Verriegelungsmechanismus (64, 88, 118) dafür ausgelegt ist, automatisch eine Verriegelungskraft auf das flexible Element (70, 94) auszuüben.

17. Anker nach einem der vorhergehenden Ansprüche, wobei die Arme (118, 138, 140) vorgespannt sind, um das flexible Element (70, 94) gegen eine Oberfläche des Lumens zu drücken.

18. Anker nach Anspruch 17, wobei die Arme (118, 138, 140) ferner dafür ausgelegt sind, das flexible Element (70, 94) freizugeben, wenn der Ankerkörper distal vorgeschoben wird.

19. Anker nach einem der vorhergehenden Ansprüche, wobei die Arme (118, 138, 140) vorgespannt sind, um in einem Winkel proximal des Ankerkörpers vorzustehen.

20. Anker nach Anspruch 19, wobei die Arme (118, 138, 140) eine sich verjüngende Öffnung definieren, durch die das flexible Element (70, 94) geführt wird.

21. Anker nach Anspruch 19, wobei die Arme (118, 138, 140) in den Ankerkörper (64, 112, 132) integriert sind.

22. Anker nach einem der vorhergehenden Ansprüche, wobei der Verriegelungsmechanismus proximal des Ankerkörpers positioniert ist.

## Revendications

1. Ancrage destiné à être implanté dans un corps, comprenant :
un corps d'ancrage (64, 112, 132) conçu pour être délivré à travers un élément creux ou un dispositif de délivrance (18) destiné à être placé contre une surface de tissu (F) ; et
un mécanisme de verrouillage (88, 118, 546) conçu pour être délivré à travers l'élément creux ou le dispositif de délivrance (18), dans lequel le mécanisme de verrouillage comprend un corps ayant une extrémité proximale et une extrémité distale, et définissant une lumière pour le passage d'un élément souple (70, 94) à travers celle-ci ;
dans lequel le mécanisme de verrouillage comprend au moins deux bras (550, 552), dans lequel un premier bras des au moins deux bras est agencé plus près de l'extrémité proximale du corps et un deuxième bras des au moins deux bras est agencé plus près de l'extrémité distale du corps, et dans lequel les au moins deux bras sont sollicités pour faire saillie dans des directions opposées jusque dans la lumière pour sangler l'élément souple (70, 94) en créant un parcours tortueux de sorte que l'élément souple (70, 94) peut être avancé de manière unidirectionnelle à travers la lumière par rapport au corps d'ancrage (64, 112, 132).

2. Ancrage selon la revendication 1 dans lequel le corps d'ancrage comprend un ancrage à panier (306).

3. Ancrage selon la revendication 2 dans lequel l'ancrage à panier (306) peut être reconfiguré d'une configuration de délivrance au sein de l'élément creux ou du dispositif de délivrance à une configuration déployée à l'extérieur de l'élément creux.

4. Ancrage selon l'une quelconque des revendications précédentes dans lequel l'élément creux ou le dispositif de délivrance est choisi parmi le groupe constitué par des endoscopes, des éléments creux, des éléments tubulaires, des cathéters, des aiguilles, et des trocarts.

5. Ancrage selon l'une quelconque des revendications précédentes dans lequel l'élément souple (70, 94) comprend une longueur de suture.

6. Ancrage selon la revendication 5 dans lequel la suture comprend une pluralité de nœuds ou de saillies (170) le long de la longueur de la suture.

7. Ancrage selon la revendication 6 dans lequel les nœuds ou saillies (170) sont formés à intervalles réguliers le long de la longueur.

8. Ancrage selon la revendication 6 dans lequel les nœuds ou saillies (170) sont formés de manière intermittente le long de la longueur.

9. Ancrage selon l'une quelconque des revendications 5 à 8, dans lequel la longueur de suture comprend un matériau de monofilament, de multifilament, élastique, ou élastomérique.

10. Ancrage selon l'une quelconque des revendications 5 à 8, dans lequel la longueur de suture comprend un matériau métallique, préférablement dans lequel le matériau métallique est choisi parmi le groupe constitué par le Nitinol, l'acier inoxydable, et le titane.

11. Ancrage selon l'une quelconque des revendications 5 à 10, dans lequel au moins une partie de la longueur de suture est entourée d'un matériau ayant un coefficient de frottement supérieur à celui de la suture.

12. Ancrage selon la revendication 11 dans lequel le matériau comprend un revêtement ou un manchon métallique (181) .

13. Ancrage selon l'une quelconque des revendications précédentes dans lequel le corps d'ancrage (64, 112, 132) définit une ouverture effilée pour le passage de l'élément souple à travers celle-ci.

14. Ancrage selon l'une quelconque des revendications précédentes dans lequel le mécanisme de verrouillage (64, 88, 118) est contenu au sein du corps d'ancrage (64, 112, 132).

15. Ancrage selon l'une quelconque des revendications précédentes dans lequel le mécanisme de verrouillage (64, 88, 118) est en ligne avec l'élément souple (70, 94).

16. Ancrage selon l'une quelconque des revendications précédentes dans lequel le mécanisme de verrouillage (64, 88, 118) est conçu pour communiquer automatiquement une force de verrouillage sur l'élément souple (70, 94).

17. Ancrage selon l'une quelconque des revendications précédentes dans lequel les bras (118, 138, 140) sont sollicités pour sangler l'élément souple (70, 94) contre une surface de la lumière.

18. Ancrage selon la revendication 17 dans lequel les bras (118, 138, 140) sont conçus en outre pour libérer l'élément souple (70, 94) quand le corps d'ancrage est avancé de manière distale.

19. Ancrage selon l'une quelconque des revendications précédentes dans lequel les bras (118, 138, 140) sont sollicités pour faire saillie à un angle de manière proximale par rapport au corps d'ancrage.

20. Ancrage selon la revendication 19 dans lequel les bras (118, 138, 140) définissent une ouverture effilée à travers laquelle l'élément souple (70, 94) est passé.

21. Ancrage selon la revendication 19 dans lequel les bras (118, 138, 140) sont d'un seul tenant avec le corps d'ancrage (64, 112, 132).

22. Ancrage selon l'une quelconque des revendications précédentes, dans lequel le mécanisme de verrouillage est positionné de manière proximale par rapport au corps d'ancrage.
